(19) Europäisches Patentamt
European Patent Office
Office européen des brevets

(11) Numéro de publication: **0 093 652**
**B1**

(12) **FASCICULE DE BREVET EUROPÉEN**

(45) Date de publication du fascicule du brevet: **17.12.86**

(21) Numéro de dépôt: **83400827.8**

(22) Date de dépôt: **26.04.83**

(51) Int. Cl.⁴: **A 61 K 37/02**, A 61 K 39/108, A 61 K 39/385, G 01 N 33/545, C 07 K 5/10, C 07 K 7/06, C 07 K 7/08

(54) **Toxine synthétique ST, son procédé de préparation et son application comme agent vaccinant.**

(30) Priorité: **26.04.82 FR 8207179**

(43) Date de publication de la demande:
**09.11.83 Bulletin 83/45**

(45) Mention de la délivrance du brevet:
**17.12.86 Bulletin 86/51**

(84) Etats contractants désignés:
**BE CH DE FR GB IT LI NL**

(56) Documents cité:
**EP-A-0 018 189**
**EP-A-0 037 522**
**FR-A-2 380 296**
**US-A-4 290 944**

(73) Titulaire: **INSTITUT PASTEUR, 28, rue du Docteur Roux, F-75715 Paris Cedex 15 (FR)**

(72) Inventeur: **Duflot, Anabela, 72, rue Jean Bleuzen, F-92170 Vanves (FR)**
Inventeur: **Gras, Hélène, 31, rue du Petit Flot, F-59510 Hem (FR)**
Inventeur: **Tartar, André, Rue du Moulin, F-62490 Vitry- en- Artois (FR)**
Inventeur: **Duflot, Edith, 18, rue Pascal, F-94230 Cachan (FR)**
Inventeur: **Boquet, Patrice, 1, rue du Puits Georget, F-94000 Creteil (FR)**

(74) Mandataire: **Gutmann, Ernest, S.C. Ernest Gutmann - Yves Plasseraud 67, boulevard Haussmann, F-75008 Paris (FR)**

EP 0 093 652 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

L'invention concerne de nouveaux peptides synthétiques, leur procédé de préparation et leur application à la production d'anticorps susceptibles de neutraliser l'activité biologique des entérotoxines stables à la chaleur, produites par les souches d'Escherichia coli.

On sait que les souches d'E. coli produisent des entérotoxines peptidiques responsables de diarrhées chez l'homme et l'animal. On a identifié au moins deux classes d'entérotoxines peptidiques dont une classe est constituée par des entérotoxines de faible poids moléculaire et stables à la chaleur, ci-après désignés par "entérotoxines ST" ou "entérotoxines ST naturelles". On sait que les entérotoxines ST se rencontrent sous des formes moléculaires multiples qui diffèrent par la taille moléculaire, la composition en acides aminés, la configuration due aux liaisons intramoléculaires, etc.

La multiplicité des formes sous lesquelles les entérotoxines sont susceptibles de se présenter, a rendu leur identification difficile, ceci d'autant plus qu'il est nécessaire de travailler sur des entérotoxines purifiées.

Les procédés de purification connus à ce jour ont permis de déterminer la séquence en acides aminés d'une entérotoxine ST provenant d'E. coli de porc (SO M. et MC CARTHY E. (1980) Proc. Natl. Acad. Sci. USA 77, 4 011-4 015) ainsi que la structure primaire d'une entérotoxine ST provenant d'E. coli humain (CHAN S. K. et GIANELLA R. A. (1981) J. Biol. Chem. 256, 7 744-7 746).

On a pu ainsi établir que la séquence peptidique de l'entérotoxine ST humaine comportait 18 acides aminés, que l'activité biologique et la toxicité sont dues à la présence de ponts disulfures et que la séquence peptidique de l'enterotoxine ST porcine ne différait de celle de l'homme que par deux acides aminés (STAPLES S. J., ASHER S. E. et GIANELLA R. A. (1980) The Journal of Biological Chemistry, 255, n° 10, 716-4 721) et (CHAN S. K. et GIANELLA R. A. (1981), The Journal of Biological Chemistry, 256, n° 15, 744-7 746).

On a également montré que les 4 premiers acides aminés de l'entérotoxine ST humaine et porcine n'étaient pas nécessaires à l'activité biologique, tandis que les quatorze derniers acides aminés de la séquence sont biologiquement actifs et responsables de la toxicité (CHAN S. K. et GIANNELLA R. A. (1981), The Journal of Biological Chemistry, 256, n° 15, 7 744-7 746).

Ce procédé de purification présente cependant l'inconvénient d'être long et complexe et ne permet d'obtenir que de très faibles quantités d'entérotoxines purifiées.

La difficulté d'obtenir des entérotoxines ST purifiées explique la raison pour laquelle la nature antigénique de l'entérotoxine ST restait à ce jour mal connue. On a longtemps cru que l'entérotoxine ST n'était pas immunogène. Contradictoirement à cette hypothèse, des travaux récents ont permis de montrer que l'entérotoxine ST, dans laquelle les ponts disulfures sont présents, est susceptible d'induire des anticorps qui fixent l'entérotoxine ST et en neutralisent l'activité bioloqique (FRANTZ J. C. et ROBERTSON D. C. (1981) Infect. and Immunity, 33, 193-198), mais que ces mêmes anticorps ne sont pas susceptibles d'établir de liaison avec une entérotoxine ST dans laquelle les ponts disulfures ont été détruits par oxydation à l'acide performique (GIANNELLA R. A., DRAKE K. W. et LUTTREL M. (1981) Infect. and Immunity 33,'186-192), de sorte que les ponts disulfures intramoléculaires paraissaient nécessaires à la conservation des propriétés immunogènes ainsi mises en évidence dans les toxines naturelles.

Or, la société demanderesse a découvert des nouveaux peptides synthétiques, sans ponts disulfures intramoléculaires, et qui à la fois témoignent d'une innocuité relative remarquable et induisent des anticorps capables d'établir des liaisons non seulement avec la séquence peptidique synthétique qui les a induites, mais de façon tout à fait inattendue et surprenante, avec l'entérotoxine ST naturelle porcine ou humaine, et en neutralisent la toxicité, en dépit du fait que l'entérotoxine ST porcine ou humaine présente des ponts disulfures intramoléculaires.

Par ponts disulfures intramoléculaires, on désigne les liaisons susceptibles de s'établir entre les atomes de soufre de deux résidus cystéyle appartenant au même enchaînement peptidique. Mais cette expression n'exclut pas la possibilité de l'existence, dans les peptides conformes à l'invention, d'une liaison disulfure entre l'atome de soufre d'un résidu cystéyle appartenant à une première chaine peptidique et l'atome de soufre d'un autre résidu cystéyle appartenant a une deuxième chaîne peptidique.

L'invention a pour but de fournir des peptides synthétiques, non toxiques, stables dans les conditions biologiques, et présentant des propriétés particulièrement intéressantes vis-à-vis des entérotoxines ST naturelles toxiques.

L'invention a pour but de fournir des peptides synthétiques, non toxiques, capables d'induire des anticorps qui reconnaissent d'une part les peptides à partir desquels ils ont été induits, et d'autre part, des entérotoxines ST naturelles humaines et animales, notamment porcines.

L'invention a également pour but de fournir des peptides synthétiques non toxiques qui, en association avec diverses molécules porteuses appropriées, sont susceptibles d'induire des anticorps qui neutralisent la toxicité des entérotoximes ST humaines et animales, motamment porcines.

L'invention a également pour but de fournir des peptides synthetiques utilisables comme déterminants antigéniques stables.

L'invention a également pour but de fournir des peptides synthétiques non toxiques qui peuvent être utilisés dans la mise en oeuvre de tests radio-immunologiques ou de tests immuno-enzymatiques, tests d'agglutination par exemple des hématies ou du latex pour détecter la présence d'entérotoxines ST humaines ou animales, notamment porcines.

L'invention a également pour but de fournir des peptides synthétiques non toxiques permettant

l'immunisation de l'homme et des animaux vis-à-vis des entérotoxines ST humaines et des animaux vis-à-vis des entérotoxines ST humaines ou animales, notamment porcines.

L'invention a pour but de fournir un procédé permettant d'obtenir directement des peptides non toxiques et par conséquent, utilisables en l'absence de tout recours nécessaire à des étapes de détoxification et de purification.

L'invention a également pour but de fournir un procédé permettant d'obtenir, en grande quantité, des peptides synthétiques de structure déterminée.

Dans l'un de ses premiers aspects, l'invention a pour objet un peptide P-(P')n comportant au plus 18(n+1) amino-acides et au moins 4(n+1) amino-acides, lévogyres, caractérisé en ce que:

- n vaut 0 ou 1,

et en ce que lorsque n vaut 0, la séquence peptidique P est la séquence suivante (3):

Asn-Thr-Phe-Tyr-Cys-Gly-Gly-Gly

ou est contenue dans l'enchaînement peptidique suivant:

Asn-Thr-Phe-Tyr-Cys-Cys-Glu-Leu-Cys-Cys-A-Pro-Ala-Cys-Ala-Gly-Cys-T(I)

(N-ter) (C-ter)

dans laquelle soit A représente Asn et T représente Tyr, soit A représente Tyr et T représente Asn;

et caractérisé en ce que les groupes thiols des éventuels résidus cystéyle présents dans la molécule ont été protégés par des groupements stables dans les conditions biologiques l'un au plus des groupes thiols pouvant être sous-forme de groupe SH libre ou pouvant être protégé par un groupe non stable dans les conditions biologiques; et en ce que lorsque n vaut 1, la séquence peptidique P-P' est constituée par deux séquences peptidiques P et P' identiques ou différentes comportant chacune au plus 18 acides aminés et au moins 4 acides aminés, contenues dans l'enchaînement peptidique de formule (I) ci-dessus indiquée, les deux séquences peptidiques P et P' étant reliées entre elles:

- par l'intermédiaire d'une liaison disulfure établie entre l'atome de soufre de l'un quelconque des résidus cystéyle de l'une des deux séquences et l'atome de soufre de l'un quelcoq ue des résidus cystéyle de l'autre séquence;

- ou par l'intermédiaire d'une liaison établie entre le groupe carboxyle de l'une des deux séquences peptidiques P et P' et le groupe amine de l'autre séquence;

et caractérisé en ce que l'un au plus des groupes thiols, s'il n'est pas engagé dans une liaison disulfure, peut être sous forme de groupe SH libre, ou protégé par un groupe non stable dans les conditions biologiques et les autres groupes thiols des éventuels résidus cystéyle sont protégés par un groupement protecteur stable dans les conditions biologiques.

Dans l'un des aspects préférés de l'invention, les séquences peptidiques P et P' sont identiques.

Dans l'un de ses aspects préférés l'invention a pour objet un peptide P-(P')n comportant au plus 18(n+1)amino-acides et au moins 4(n+1) amino-acides, caractérisé en ce que:

- n vaut 0 lorsque la séquence peptidique P ne comporte aucun résidu cystéyle;

- n vaut 0 ou 1, lorsque la séquence peptidique P comporte au moins un résidu cystéyle;

et en ce que, lorsque n vaut 0, la séquence peptidique P est la séquence suivante (3):

Asn-Thr-Phe-Tyr-Cys-Gly-Gly-Gly

ou est contenue dans l'enchaînement peptidique suivant:

Asn-Thr-Phe-Tyr-Cys-Cys-Glu-Leu-Cys-Cys-A-Pro-Ala-Cys-Ala-Gly-Cys-T(I)

(N-ter) (C-ter)

dans lequel soit A représente Asn et T représente Tyr, soit A représente Tyr et T représente Asn;

et caractérisé en ce que les groupes thiols des éventuels résidus cystéyle présents dans la molécule ont été protégés par des groupements stables dans les conditions biologiques, l'un au plus des groupes thiols pouvant être sous forme de groupe SH libre ou pouvant être protégé per un groupe non stable dans les conditions biologiques; et en ce que lorsque n vaut 1, la séquence peptidique P-P' est constituée par deux séquences peptidiques P et P' identiques comportant chacune au plus 18 acides aminés et au moins 4 acides aminés, contenues dans l'enchaînement peptidique de formule (I) ci-dessus indiquée, les deux séquences peptidiques P et P' étant reliées entre elles par l'intermédiaire d'un liaison disulfure établie entre l'atome de soufre de l'un quelconque des résidus cystéyle de l'une des deux séquences et l'atome de soufre de l'un quelconque des résidus cystéyle de l'autre séquence, et caractérisé en ce que les autres groupes thiols, éventuellement présents, non engagés dans la liaison disulfure des éventuels résidus cystéyle sont protégés per un groupement protecteur stable dans les conditions biologiques.

D'une façon générale, l'invention concerne tout peptide contenant une ou plusieurs séquences P et P' telles qu'elles ont été définies précédemment et susceptibles d'induire in vivo des anticorps contre les entérotoxines ST, ces différentes séquences P et P' pouvant d'ailleurs être reliées entre elles per des groupes de pontage, eux-mêmes peptidiques ou non, dans la mesure où ces groupes de pontage n'interfèrent pas avec les qualités immunogènes attribuables aux déterminants antigéniques que contiennent les séquences P et P' en question.

D'une façon générale, l'invention concerne tout peptide répondant à la susdite condition qui, le cas échéant, après fixation sur un support macromoléculaire approprié, est capable d'induire in vivo la production d'anticorps actifs contre les peptides tels qu'ils ont été définis plus haut, en rapport avec l'un des premiers aspects de l'invention, ou plus particulièrement encore contre l'entérotoxine ST naturelle.

A titre d'exemple de ponts peptidiques, on peut mentionner des oligomères de L-lysine, contenant par exemple jusqu'à 10 unités de lysine, de préférence 5, ou encore par exemple des groupes dérivés d'adipimate

reliant des fonctions amines portées per des séquences peptidiques P et P' distinctes.

D'une façon générale, on peut avoir recours, pour la constitution de tous ces groupes de pontage, à toute molécule comportant des groupes fonctionnels, tels qu'amine ou carboxylique, susceptibles de réagir avec des fonctions respectivement carboxylique et amine appartenant à des séquences P distinctes. D'autres groupes de pontage peuvent précisément également faire intervenir des groupes SH entièrement libres des séquences P sus-définies, lorsqu'elles les contiennent.

Le terme peptide englobe dans la suite les peptides qui seront désignés par peptides monomères et ceux qui seront designes par peptides dimères.

Les peptides monomères correspondent au cas où n est égal à 0, c'est-à-dire à un enchaînement peptidique dans lequel chaque résidu amino-acyle est engagé avec le ou les résidus amino-acyle adjacents par une ou des liaisons peptidiques.

Les peptides dimères correspondent au cas où n est égal à 1, c'est-à-dire au cas où le peptide comporte deux enchaînements peptidiques identiques, dans lesquels chacun des amino acyles est engagé, avec les deux amino acyles adjacents par une liaison peptidique, ces deux enchaînements peptidiques étant reliés entre eux par l'intermédiaire d'une liaison disulfure établie entre l'atome de soufre d'un résidu cystéyle quelconque de l'un des enchaînements avec l'atome de soufre d'un résidu cystéyle quelconque de l'autre enchaînement.

Par groupes non stables dans les conditions biologiques, on désigne les groupements protecteurs dont la fixation aux groupes thiols est réversible. En d'autres termes, ces groupes non stables ne restent fixés sur les groupes thiols qu'à partir d'une concentration suffisante. Au-dessous de cette concentration, ces groupes non stables se dissocient des groupes thiols. La concentration limite à partir de laquelle les groupes non stables se dissocient dépend de la nature des réactions, des conditions de reaction et des composés contenant lesdits groupes thiols a protéger. A titre d'exemple de groupes non stables dans les conditions biologiques on peut citer le betamercaptoéthanol, le dithiothreitol, le chlorure de mercure, le parachloromercurobenzène, le 4,4'-dithio-dipyridine.

Par groupe stable dans les conditions biologiques, on désigne les groupements protecteurs qui après avoir été fixés aux groupes thiols, y restent fixés même lorsque les peptides sont mis en présence d'un milieu biologique, en dépit des réactions métaboliques susceptibles d'intervenir ou toute autre réaction pouvant modifier les caractéristiques du peptide. Ces groupes protecteurs empêchent ainsi la formation de ponts disulfures intramoléculaires qui, s'ils se formaient, conféreraient au peptide des propriétés toxiques.

Dans les peptides selon l'invention, tout groupement protecteur de la fonction thiol, dans la mesure où il est stable dans les conditions biologiques, convient.

A titre d'exemples de groupes protecteurs de la fonction thiol et stables dans les conditions biologiques, on peut citer ceux qui sont mentionnés dans les pages 137 à 164 de l'article intitulé "Sulfhydryl group protection in peptide synthesis" de R. G. HISKEY, The Peptides, vol. 3 (1981), et dans les pages 233 à 247 de l'article intitulé (Solid phase peptide synthesis" de G. BARANY et R. B. MERRIFIELD, The Peptides, vol. 3 (1981).

A titre de groupes protecteurs, on peut également avoir recours aux groupes qui sont utilisés en biochimie pour la modification définitive des fonctions thiol sur les protéines.

Dans la pratique, il faut que le groupement protecteur de la fonction thiol soit compatible avec les conditions de synthèse utilisées pour l'obtention des peptides selon l'invention et en particulier soit stable dans les conditions de synthèse, y compris celle de la déprotection finale.

En effet, plusieurs paramètres interdépendants sont à prendre en considération dans le choix du groupe protecteur de la fonction thiol, puisque le type même de la synthèse mis en oeuvre détermine notamment la nature des groupes de protection des fonctions acide et amine à utiliser, qui eux-mêmes déterminent l'agent déprotecteur final.

Dans la pratique, on a avantageusement recours aux groupes suivants pour protéger la fonction thiol:

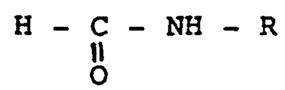

Dans ce cas, la fonction thiol protégée peut être représentée comme suit:

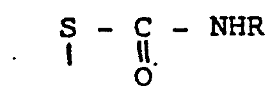

A titre de groupes protecteurs de la fonction thiol, sont avantageux les composés de formule suivante:

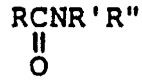

dans laquelle R, R' et R'' représentent indépendamment les uns des autres un atome d'hydrogène, un radical alcoyle de 1 à 4 atomes de carbone.

Parmi ces composés, les composés de formule:

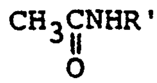 , 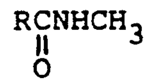 , 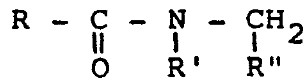

dans lesquels R et R' représentent un atome d'hydrogène, un radical alcoyle ayant de 1 à 4 atomes de carbone sont avantageux.

Les fonctions thiol protégées par chacun des trois groupes protecteurs ci-dessus indiqués peuvent alors respectivement être représentées par:

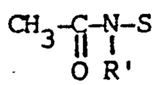 , 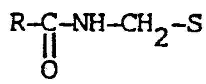 , 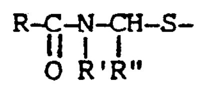

Dans la pratique, il est particulièrement avantageux d'utiliser l'acétamidométhyle ou le formamideméthyle. Ces deux groupements protecteurs de la fonction thiol sont stables dans les conditions biologiques et présentent l'avantage de ne pas être déprotégés par les réactions de déprotection finale généralement utilisés et qui servent d'une part à séparer le peptide synthétique du support sur lequel il a été généralement préparé d'une part, à éliminer les groupes de protection des fonctions acides et amines généralement utilisés au cours de la synthèse.

Une classe préférée de peptides selon l'invention ci-après désignée par GI, est constituée per les peptides monomères.

Ces peptides monomères selon l'invention comportent au plus 18 amino-acides et au moins 4 amino-acides et sont caractérisés en ce que leur enchaînement peptidique est la séquence suivante (3):

Asn-Thr-Phe-Tyr-Cys-Gly-Gly-Gly

ou est contenu dans la séquence peptidique suivante:

Asn-Thr-Phe-Tyr-Cys-Cys-Glu-Leu-Cys-Cys-A-Pro-Ala-Cys-Ala-Gly-Cys-T

(N-ter) (C-ter)

dans laquelle, soit A représente Asn et T représente Tyr, soit A représente Tyr et T représente Asn; et

en ce que les groupes thiols des éventuels résidus cystéyle présents dans la molécule sont protégés par des groupements stables dans les conditions biologiques, l'un au plus des groupes thiols pouvant être sous forme de groupe SH libre ou pouvant être protégé par um groupe non stable dans les conditions biologiques.

Parmi la classe G1, une classe de peptides monomères préférés selon l'invention est constituée par ceux dans lesquels tous les groupes thiols des éventuels résidus cystéyle ont été protégés par des groupements stables dans les conditions biologiques. Cette classe sera dans la suite désignée par G1A.

Une autre classe de peptides monomères préférés selon l'invention est constituée par ceux dans lesquels tous les groupes thiols des éventuels résidus cystéyle, sauf un, ont été protégés par des groupements stables dans les conditions de déprotection. Cette classe de monopeptides sera dans la suite désignée par G1B.

Dans le cas des peptides appartenant à G1B, le seul groupe thiol qui n'a pas été protégé par un groupement stable dans les conditions biologiques, peut être à l'état de groupe SH libre (ou peut avoir été protégé par un groupe non stable dans les conditions biologiques).

Lorsque le groupement est non stable dans les conditions de la déprotection, au cours de cette étape, le groupement protecteur est éliminé et le groupe thiol se retrouve alors à l'état de groupe SH libre ou de disulfure mixte.

A titre d'exemples de groupes protecteurs du groupe SH susceptibles de se libérer au cours de la déprotection, on peut citer le groupement paraméthoxybenzylique ou le groupement S-tertiobutylsulfényle.

Dans les chaînes peptidiques indiquées ciaprès, à titre d'exemple, il est entendu que l'aminoacyle d'extrémité à la gauche des séquences indiquées, est un amino-acyle N-terminal et l'amino-acyle d'extrémité à

la droite de la formule, un amino-acyle C-terminal, sauf lorsqu'il sera spécifié autrement. Par exemple, Asn-Thr-Phe-Tyr correspond à la chaîne peptidique dans laquelle Asn est le groupe N-terminal et Tyr est le groupe C-terminal.

Parmi les classes G1, G1A et G1B, une classe préférée de peptides selon l'invention est constituée par les peptides qui comprennent l'une des séquences suivantes:

- Asn-Thr-Phe-Tyr
- Asn-Thr-Phe-Tyr-Cys
-Cys-Cys-Asn-Pro-Ala-Cys
-Cys-Cys-Tyr-Pro-Ala-Cys
-Asn-Thr-Phe-Tyr-Cys-Cys-Glu
-Asn-Thr-Phe-Tyr-Cys-Cys-Glu-Leu
-Cys-Cys-Gly-Leu-Cys-Cys-Asn-Pro-Ala-Cys-Ala-Gly-Cys
-Cys-Cys-Gly-Leu-Cys-Cys-Tyr-Pro-Ala-Cys-Ala-Gly-Cys

A l'intérieur de cette classe ci-dessus définie, une sous-classe de peptides préférés selon l'invention est constituée par ceux qui répondent à la formule suivante:

Asn-Thr-Phe-Tyr
Asn-Thr-Phe-Tyr-Cys
Cys-Cys-Asn-Pro-Ala-Cys
Cys-Cys-Tyr-Pro-Ala-Cys
Asn-Thr-Phe-Tyr-Cys-Cys-Glu
Asn-Thr-phe-Tyr-Cys-Cys-Glu-Leu
Cys-Cys-Gly-Leu-Cys-Cys-Asn-Pro-Ala-Cys-Ala-Gly-Cys
Cys-Cys-Gly-Leu-Cys-Cys-Tyr-Pro-Ala-Cys-Ala-Gly-Cys

Les trois peptides suivants sont particulièrement préférés:

Asn-Thr-Phe-Tyr-Cys-Cys-Gly-Leu-Cys-Cys-Asn-Pro-Ala-Cys-Ala-Gly-Cys-Tyr (1)
Asn-Tyr-Phe-Tyr-Cys-Cys-Gly-Leu-Cys-Cys-Tyr-Pro-Ala-Cys-Ala-Gly-Cys-Asn (2)
Asn-Thr-Phe-Tyr-Cys-Gly-Gly-Gly (3)

Une autre classe de peptides selon l'invention ci-après désignée par G2 est constituée par les dipeptides ci-dessus définis dans lesquels n vaut 1.

Ces dipeptides P-P' selon l'invention comportent au plus 18 x 2 acides aminés et au moins 4 x 2 acides aminés et sont caractérisés par le fait qu'ils sont constitués par deux séquences peptidiques P et P' identiques comportant chacune au plus 18 acides aminés et au moins 4 acides aminés, contenues dans l'enchaînement peptidique de formule (I) ci-dessus indiquée, les deux séquences peptidiques Pet P' étant reliées entre elles par l'intermédiaire d'une liaison disulfure établie entre l'atome de soufre de l'un des résidus cystéyle de l'une des deux séquences peptidiques et l'atome de soufre de l'un des résidus cystéyle de l'autre séquence peptidique, et caractérisés en ce que les autres groupes thiols -non engagés dans la liaison disulfure- des éventuels résidus cystéyle sont protégés par un groupement protecteur stable dans les conditions biologiques.

A l'intérieur de cette classe G2 de composés ci-dessus definis, une sous-classe préferée de peptides dimères selon l'invention est constituée per ceux dans lesquels la liaison disulfure entre les deux séquences peptidiques P et P' est établie entre deux résidus cystéyle, occupant les mêmes positions sur chacune des séquences peptidiques P et P'.

A l'intérieur de la classe G2 des peptides dimères P-P' selon l'invention, une classe préferée est constituée par ceux dans lesquels les séquences peptidiques P et P' comprend l'un des enchaînements peptidiques suivants:

-Asn-Thr-Phe-Tyr-Cys
-Cys-Cys-Asn-Pro-Ala-Cys
-Cys-Cys-Tyr-Pro-Ala-Cys
-Asn-Thr-Phe-Tyr-Cys-Cys-Glu
-Asn-Thr-Phe-Tyr-Cys-Cys-Glu-Leu
-Cys-Cys-Gly-Leu-Cys-Cys-Asn-Pro-Ala-Cys-Ala-Gly-Cys
-Cys-Cys-Gly-Leu-Cys-Cys-Tyr-Pro-Ala-Cys-Ala-Gly-Cys

A l'intérieur de cette sous-classe, une classeaventageuse de peptides dimères P-P' selon l'invention est constituée par ceux dans lesquels la séquence peptidique de P et P' repond à la formule suivante:

Asn-Thr-Phe-Tyr-Cys
Asn-Thr-Phe-Tyr-Cys-Gly-Gly-Gly (3)
Cys-Cys-Asn-Pro-Ala-Cys
Cys-Cys-Tyr-Pro-Ala-Cys
Asn-Thr-Phe-Tyr-Cys-Cys-Glu
Asn-Thr-Phe-Tyr-Cys-Cys-Glu-Leu
Cys-Cys-Gly-Leu-Cys-Cys-Asn-Pro-Ala-Cys-Ala-Gly-Cys
Cys-Cys-Gly-Leu-Cys-Cys-Tyr-Pro-Ala-Cys-Ala-Gly-Cys.
Asn-Thr-Phe-Tyr-Cys-Cys-Gly-Leu-Cys-Cys-Asn-Pro-Ala-Cys-Ala-Gly-Cys-Tyr (1)
Asn-Thr-Phe-Tyr-Cys-Cys-Gly-Leu-Cys-Cys-Tyr-Pro-Ala-Cys-Ala-Gly-Cys-Asn (2)

Des peptides dimères particulièrement appropriés selon l'invention ont pour formule, par exemple selon le modèle:

Cys-Cys-Asn-Pro-Ala-Cys
Cys-Cys-Asn-Pro-Ala-Cys
Cys-Cys-Asn-Pro-Ala-Cys
Cys-Cys-Asn-Pro-Ala-Cys
Asn-Thr-Phe-Tyr-Cys-Cys-Gly-Leu-Cys-Cys-Asn-Pro-Ala-Cys-Ala-Gly-Cys-Tyr
Asn-Thr-Phe-Tyr-Cys-Cys-Gly-Leu-Cys-Cys-Asn-Pro-Ala-Cys-Ala-Gly-Cys-Tyr
Asn-Thr-Phe-Tyr-Cys-Cys-Gly-Leu-Cys-Cys-Tyr-Pro-Ala-Cys-Ala-Gly-Cys-Asn
Asn-Thr-Phe-Tyr-Cys-Cys-Gly-Leu-Cys-Cys-Tyr-Pro-Ala-Cys-Ala-Gly-Cys-Asn

L'invention concerne également les conjugués comportant un ou plusieurs peptides selon l'invention liés de façon covalente à une molécule porteuse, physiologiquement acceptable et non toxique.

Les molécules porteuses entrant dans la constitution des conjugués selon l'invention sont de préférence choisies parmi les molécules biologiques ayant un site d'action au niveau de l'intestin.

A titre d'exemples de protéines naturelles, on peut citer la toxine tétanique, l'ovalbumine, et les sérums albumines.

A titre de supports macromoléculaires synthétiques, on mentionnera par exemple des polylysines ou des poly(D-L-alanine)-poly(L-lysine)

La littérature mentionne d'autres types de supports macromoléculaires susceptibles d'être utilisés, lesquels présentent en général un poids moléculaire supérieur à 20 000.

A titre de molécule porteuse particulièrement avantageuse, on peut avoir recours à une protéine porteuse elle-même immunogène, pour autant que les immunogénécités ainsi conférées à l'ensemble ne se nuisent pas mutuellement.

Un exemple de protéine porteuse appropriée est la cytotoxine de Shigella (cf. The Journal of Biological Chemistry, vol. 256, n° 16, August, 25, 1981, p. 8 732-8 738) ou des fragments de cytotoxine de Shigella comportant des déterminants antigéniques essentiels de la cytotoxine de la dysenterie.

Un autre exemple de protéine porteuse particulièrement appropriée est le choléragénoïde ou des fragments de choléragénoïde comportant des déterminants antigéniques essentiels de la toxine du choléra.

Le choléragénoïde, agrégat non toxique de la sous-unité B de la toxine du choléra, peut être purifié selon la méthode décrite dans Infection and Immunity, June 1977, p. 789-795.

L'avantage du choléragénoïde est qu'il contient les déterminants antigéniques principaux de la toxine du choléra et se fixe à l'endroit approprié, à savoir sur la muqueuse intestinale pour qu'il y ait immunité efficace.

Par conséquent, le conjugué moléculaire constitué par le choléragénoïde, en association avec l'un quelconque des peptides selon l'invention semble particulièrement bien approprié en raison du fait que chacun des eléments du conjugué présente une activité immunogène locale se manifestant au même endroit, à savoir au niveau de l'intestin.

Une classe préférée de conjugués selon l'invention est constituée par ceux dans lesquels la molécule porteuse est le choléragénoïde ou la cytotoxine de Shigella et le peptide selon l'invention comprend la séquence suivante:

-Asn-Thr-Phe-Tyr
-Asn-Thr-Phe-Tyr-Cys
-Cys-Cys-Asn-Pro-Ala-Cys
-Cys-Cys-Tyr-Pro-Ala-Cys
-Asn-Thr-Phe-Tyr-Cys-Cys-Glu
-Asn-Thr-Phe-Tyr-Cys-Cys-Glu-Leu
-Cys-Cys-Gly-Leu-Cys-Cys-Asn-Pro-Ala-Cys-Ala-Gly-Cys
-Cys-Cys-Gly-Leu-Cys-Cys-Tyr-Pro-Ala-Cys-Ala-Gly-Cys

Une autre classe avantageuse de conjugués selon l'invention est constituée par ceux dans lesquels la molécule porteuse est le choléragénoïde ou la cytotoxine de Shigella et le peptide selon l'invention répond à la formule suivante:

Asn-Thr-Phe-Tyr
Asn-Thr-Phe-Tyr-Cys
Asn-Thr-Phe-Tyr-Cys-Gly-Gly-Cly

Cys-Cys-Asn-Pro-Ala-Cys
Cys-Cys-Tyr-Pro-Ala-Cys
Asn-Thr-Phe-Tyr-Cys-Cys-Glu
Asn-Thr-Phe-Tyr-Cys-Cys-Glu-Leu
Cys-Cys-Gly-Leu-Cys-Cys-Asn-Pro-Ala-Cys-Ala-Gly-Cys
Cys-Cys-Gly-Leu-Cys-Cys-Tyr-pro-Ala-Cys-Ala-Gly-Cys
Asn-Tyr-Phe-Tyr-Cys-Cys-Gly-Leu-Cys-Cys-Asn-Pro-Ala-Cys-Ala-Cys-Tyr
Asn-Tyr-Phe-Tyr-Cys-Cys-Gly-Leu-Cys-Cys-Tyr-Pro-Ala-Cys-Ala-Cys-Asn

## SYNTHESE DES PEPTIDES SELON L'INVENTION

On peut avoir recours à des procédés connus en soi, pour effectuer la synthèse des monopeptides selon l'invention. Ces procédés sont résumés ci-après.

La synthèse des peptides en solution homogène et en phase solide est bien connue.

A cet égard, on pourra avoir recours au mode de synthèse en solution homogène décrit par HOUBEN WEYL dans l'ouvrage intitulé "Methoden der Organischen Chemie" (Méthode de la chimie organique) édité par E. Wünsch., vol. 15-I et II, THIEME, Stuttgart 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide. Lorsque l'aminoacyle mis en oeuvre possède une fonction amine supplémentaire (cas de la lysine par exemple) ou une autre fonction acide (cas par exemple de l'acide glutamique), ces fonctions seront par exemple protégées, par des groupes carbobenzoxy ou t-butyloxycarbonyle, en ce qui concerne les fonctions amine, ou par des groupes t-butylester, en ce qui concerne les fonctions carboxyliques. Il en ira de même de la protection de toute autre fonction réactive. Par exemple, lorsque l'un des aminoacyles considerés contient une fonction SH (par exemple le cystéine), on pourra avoir recours à un groupe acétamidométhyle ou paraméthoxybenzyle.

Dans le cas de la synthèse progressive, acide aminé par acide aminé, la synthèse débute de préference par la condensation de l'amino-acide C-terminal avec l'amino-acide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier aminoacide C-terminal de la chaîne. Cet amino-acide est fixé sur la résine par l'intermédiaire de son groupe carboxylique et sa fonction amine est protégée, par exemple par le groupe t-butyloxycarbonyle.

Lorsque le premier amino-acide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième amino-acide qui fournit le second amino-acyle de la séquence recherchée, à partir du résidu amino-acyle C-terminal sur la fonction amine déprotégée du premier amino-acide C-terminal fixé sur la chaîne. De préference, la fonction carboxyle de ce deuxième amino-acide est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux aminoacides, et dont la fonction amine terminale est protégée. Comme précédemment, on déprotège la fonction amine et on peut ensuite procéder à la fixation du troisième aminoacyle, dans les conditions analogues à celles de l'addition du deuxième aminoacide C-terminal.

On fixe ainsi, les uns après les autres, les acides aminés, qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

Pour synthétiser les peptides dimères selon l'invention, on peut avoir recours aux peptides monomères comportant respectivement un résidu cystéyle dans lequel le groupe thiol est à l'état de SH non protégé, notamment par oxydation au sein d'un milieu contenant les monopeptides selon l'invention, par exemple à l'aide d'oxygène moléculaire. Ce milieu consiste par exemple en une solution aqueuse de pH d'environ 7.

Cette oxydation permet à la liaison disulfure de s'établir entre les atomes de soufre des résidus cystéyle comportant le groupe thiol à l'état de SH non protégé.

Pour préparer les peptides dimères selon l'invention, on peut également avoir recours aux indications de synthèse proposées aux pages 145 à 149, de l'article intitulé (Sulfhydryl group protection in peptide synthesis" de R. G. HISKEY, The Peptides, vol. 3 (1981) et aux pages 240 à 243, de l'article intitulé "Solid phase peptide synthesis", de G. BARANY et R. B. MERRI-FIELD, The Peptides, vol. 3 (1981).

Dans la pratique, on peut opérer comme suit ou de façon équivalente.

Après déprotection des peptides selon l'invention, on fait barbotter l'air dans la solution du peptide jusqu'à disparition des fonctions thiol libres.

Pour synthétiser les conjugués selon l'invention, on peut avoir recours à des procédés connus en soi, tel que celui décrit par FRANTZ et ROBERTSON dans Infect. and Immunity, 33, 193-198 (1981), ou celui décrit dans Applied and Environmental Microbiology, octobre 1981, vol. 42, n° 4, 611-614 par P. E. KAUFFMAN en utilisant le peptide et la molécule porteuse appropriée.

Dans la pratique, on utilisera avantageusement comme agent de couplage les composés suivants, cités à titre non limitatif: aldéhyde glutarique, chloroformiate d'éthyle, carbodiimides hydrosolubles [N-éthyl-N"(3-diméthylamino-propyl) carbodiimide, HCL], diisocyanates, bis-diazobenzidine, di- et trichloro-s-triazines, bromures de cyanogène, benzoquinone, ainsi que les agents de couplage mentionnés dans Scand. J. Immunol., 1978, vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives des molécules supports. Avantageusement, il s'agit des fonctions carboxyle et amine, lesquelles peuvent donner lieu a une reaction de couplage en présence d'un agent de couplage du genre de ceux utilisés dans la synthèse des protéines, par exemple, le 1-éthyl-3-(3-diméthylaminopropyl)-carbodiimide et le N-hydroxybenzotriazole. On peut encore avoir recours à la glutaraldéhyde, notamment lorsqu'il s'agit de relier entre eux des groupes aminés respectivement portés par le peptide et la molécule support.

Les exemples qui suivent concernent des synthèses de peptides préférés selon l'invention, visant à en donner une meilleure illustration, sans pour autant être limitatifs.

**EXEMPLE 1**

**SYNTHESE DU PEPTIDE MONOMERE DE FORMULE**

(1)

Asn-Thr-Phe-Tyr-Cys-Cys-Glu-Leu-Cys-Cys-Asn-Pro-Ala-Cys-Ala-Gly-Cys-Tyr

(N-ter) (C-ter)

Ce peptide monomère synthétique présente la même séquence peptidique que l'entérotoxine ST de porc. Mais à la différence de l'entérotoxine ST naturelle de porc, dans ce monopeptide selon l'invention, tous les groupes thiols des résidus cystéyle sont protégés par l'acétamidométhyle et il n'y a pas de pont disulfure intromoléculaire. Dans la suite, on désignera ce monopeptide par "Entérotoxine ST synthétique de porc".

Pour préperer ce peptide monomère de formule (1), on a recours à la synthèse peptidique mentionnée ci-dessus et on procède comme suit ou de façon équivalente.

Les abréviations utilisées dans le cadre de cette synthèse ont la signification suivante:

BOC: t-butyloxycarbonyl.
Asp: acide aspartique
Thr: thréonine
Glu: acide glutamique
Pro: proline
Gly: glycine
Ala: alanine
Asn: asparagine
Cys: cystéine
Leu: leucine
Tyr: tyrosine
Phe: phénylalanine.

On utilise comme amino-acides des N-alphaamino-acides protégés uniquement avec le groupe t-butyloxycarbonyl (BOC).

Les groupes fonctionnels latéraux sont protégés comme suit:

- la tyrosine est protégée par le 2,6-dichlo-robenzyle,
- la cystéine est protégée par l'acétamidométhyle,
- l'acide glutamique et la thréonine sont protégés par le benzyle.

On distille $CH_2Cl_2$ à partir de $Na_2CO_3$ anhydre avant utilisation.

Il est avantageux d'utiliser un support de resine constitué par un copolymère choromethylé de styrène et de divinylbenzène à 1 % (commercialisé par les laboratoires BIORAD).

La N-alpha-t-butyloxycarbonyl-0-2,6-dichloro-benzyltyrosine est de préférence estérifiée sous forme de son sel de césium (GISIN B.F. (1973) Helv. Chim. Acta 56, 1476).

La synthèse est effectuée dans un synthétiseur automatique du type de celui commercialisé par BECKMAN sous la désignation 990 B.

Chacun des acides aminés entrant dans la constitution du peptide monomère (1) selon l'invention, est fixé à la chaîne peptidique déjà formée comme il est indiqué ci-après.

a) On lave trois fois la résine pendant environ 3 minutes avec du chlorure de méthylène pour la mettre en suspension.

b) On lave ensuite une fois pendant environ trois minutes avec de l'acide trifluoroacétique à 40 % pour imprégner la résine.

c) On relave une fois pendant environ 30 minutes avec de l'acide trifluoroacétique pour déprotéger le groupe N-terminal de la chaîne peptidique déjà formée.

d) On lave ensuite deux fois pendant environ 3 minutes avec du chlorure de méthylène pour éliminer l'acide trifluoroacétique.

e) puis on lave deux fois pendant environ 3 minutes avec de l'alcool isopropylique pour éliminer le chlorure de méthylène.

f) puis on réimprègne le milieu en lavant 4 fois pendant 3 minutes avec du chlorure de méthylène.

g) On lave trois fois pendant environ 3 minutes avec de la diisopropyléthylamine pour neutraliser la fonction amine, qui se trouvait salifiée sous forme de son sel avec l'acide trifluoroacétique.

h) On lave quatre fois pendant environ 3 minutes avec du chlorure de méthylène pour chasser la diisopropylamine en excès.

i) On ajoute l'acide aminé à fixer sur la chaîne peptidique déjà formée, comme indiqué ci-dessous.

j) On lave ensuite trois fois pendant environ 3 minutes avec du chlorure de méthylène.

k) Puis on chasse le chlorure de méthylène en lavant deux fois pendant environ 3 minutes avec de l'alcool isopropylique.

l) On lave enfin trois fois pendant environ 3 minutes avec du chlorure de méthylène, pour remettre le milieu réactionnel dans son solvant.

En ce qui concerne la fixation de chacun des acides aminés à la chaîne peptidique déjà formée, on a recours à un acide aminé (en excès par rapport à la chaîne peptidique déjà formée, cet excès correspondant à environ trois fois la charge de la chaîne peptidique) dont la fonction amine est protégée par le groupe t-butyloxycarbonyle et dont la fonction acide est activée par la dicyclohexylcarbodiimide, l'hydroxybenzotriazole ensuite additionné afin de minimiser les réactions secondaires (ARENDT A., KOLODZIEJZKYK A. M. (1978) Tetrahedron Lett 40, 3867), (MOJSOv S., MITCHELL A. R. (1980) J. Org. Chem. 45, 555).

Après chaque étape d'addition d'un acide aminé, on détermine la quantité d'acide aminé libre qui n'a pas été fixée sur la chaîne peptidique, par le test à la ninhydrine (KAISER E., COLESCOTT R. L. et al. (1976) Anal. Biochem. 34, 595), sauf lorsqu'on fixe l'asparagine (en position 11 dans le peptide (1)) sur la proline (en position 12 dans le peptide (1)), la numérotation étant effectuée de gauche à droite. Lorsqu'on fixe ces deux acides aminés, on a en effet recours au test à la chloranile (CHRISTENSEN T. (1979) Acta. Chem. Scand., 833, 763). A la fin de la synthèse, on élimine tous les groupes protecteurs fixés sur le peptide porté par la résine, à l'exception du groupe acétamidométhyle qui est stable vis-à-vis de ce réactif (VEBER et al. (1972), J. Am. Chem. Soc. 94, 5456). On détache le peptide de la résine à l'aide d'acide fluorhydrique anhydre à raison de 10 ml d'HF anhydre par gramme de résine, en présence d'anisol (1 ml/g) à la température d'environ 0°C pendant environ 60 minutes.

Après évaporation de l'acide fluorhydrique, le mélange réactionnel est lavé à l'éther et le peptide est séparé de la résine par extraction à l'aide d'acide acétique à 50 %. Les extraits sont dilués à l'eau et lyophilisés.

Le peptide brut obtenu est faiblement soluble dans l'eau. Il est purifié par chromatographie sur une colonne du type de celle commercialisée sous le nom LH 20 en utilisant comme phase mobile le mélange diméthylformamide-acide acétique 0,1 M (3/1). On mesure une absorption à 254 nm. Les différentes fractions du pic principal sont examinées sur le plan de leur homogénéité et rassemblées.

On obtient le peptide purifié (97 mg, rendement global d'environ 10 %) présentant la composition en acides aminés suivante:

| | Valeur expérimentale | Valeur théorique |
|---|---|---|
| Asp° | 2,22 | 2 |
| Thr | 0,85 | 1 |
| Glu | 1,13 | 1 |
| Pro | 1,21 | 1 |
| Gly | 1,20 | 1 |
| Ala | 2,07 | 2 |
| Cys | 4,68 | 6 |
| Leu | 1,13 | 1 |
| Tyr | 2,27 | 2 |
| Phe | 1,12 | 1 |

° La valeur de l'asparagine (Asn) est donnée en Asp car au cours de l'hydrolyse, la fonction amide primaire de Asn est coupée, ce qui la transforme en Asp.

On mesure la pureté par chromatographie liquide sous haute pression en phase inversée sur une colonne du type de celle commercialisée sous le nom µ Bondapack ® C18 dans laquelle l'éluant est le mélange de solvants suivants:

| | |
|---|---|
| $CH_3OH$ | 425 ml |
| $H_2O$ | 525 ml |
| Tampon phosphate 0,05 M pH 2,5 | 50 ml |

avec contrôle à 210, 254 et 270 nm.

**EXEMPLE 2**

**SYNTHESE DU PEPTIDE MONOMERE DE FORMULE (2)**

Asn-Thr-Phe-Tyr-Cys-Cys-Glu-Leu-Cys-Cys-Tyr-Pro Ala-Cys-Ala-Gly-Cys-Asn
(N-ter) (C-ter)

Le peptide monomère de séquence ci-dessus indiquée et dans lequel tous les groupes thiols sont modifiés par l'acétamidométhyle a été préparé selon le procédé ci-dessus indiqué.

Dans la suite, ce peptide sera désigné par "Entérotoxine ST synthétique humaine", car il présente la même séquence d'acides aminés que l'entérotoxine ST naturelle humaine. Mais à la différence de l'entérotoxine ST naturelle humaine, dans le peptide selon l'invention, tous les groupes thiols des résidus cystéyle sont protégés par l'acétamidométhyle, et il n'y a pas de pont disulfure intramoleculaire.

**EXEMPLES III A VI**

Ils concernent la préparation de quatre conjugués selon l'invention dans lesquels le peptide est soit l'entérotoxine ST synthétique de porc, soit l'entérotoxine ST synthétique humaine, et la molécule porteuse est soit la toxine tétanique, soit l'ovalbumine.

On désignera dans la suite par ST-TT les conjugués dans lesquels le peptide selon l'invention est l'entérotoxine ST synthétique (de porc ou humaine) et la molécule porteuse est la toxine tétanique et par ST-OVA les conjugués dans lesquels le peptide selon l'invention est l'entérotoxine ST synthétique (de porc ou humaine) et la molécule porteuse est l'ovalbumine.

La synthèse de ces conjugués peut être effectuée selon la méthode décrite par FRANTZ et ROBERTSON dans Infect. and Immunity, 33, 193-198 (1981).

Le mélange réactionnel contient un excès d'entérotoxine ST (porcine ou humaine) synthétique correspondant à environ deux fois plus d'entérotoxine ST synthétique que de protéine porteuse. Le couplage entre l'entérotoxine ST synthétique et les différentes protéines porteuses est effectué en utilisant par exemple la 1-éthyl-3-(3-diméthylaminopropyl)-carbodiimide. Le mélange réactionnel est mis à incuber dans l'obscurité pendant environ 18 heures à température ambiante.

On a ensuite recours à une dialyse extensive effectuée sur un tampon PiNa, à pH 7,00, pour éliminer l'entérotoxine ST synthétique libre et l'agent de couplage qui n'a pas réagi.

Le tampon PiNa est un tampon phosphate de sodium dont la composition pour un litre d'eau distillée est la suivante:

| | | | |
|---|---|---|---|
| NaCl...... | 8 g | $NaHPO_4 \cdot 12H_2O$.... | 2,8 g |
| $KH_2PO_4$.... | 0,2 g | KCl............. | 0,2 g |

Les conjugues ST-OVA peuvent être utilisés directement pour effectuer les essais d'immunisation sur les animaux.

Dans le cas des conjugués ST-TT, il est nécessaire de détoxifier la préparation de l'aide de formaldéhyde selon la méthode décrite par BLASS et al. dans Bull. Soc. Chim. 10, 3057-3965 (1967).

Avant d'utiliser le conjugué ST-TT selon l'invention, on détermine sa toxicité par injection intramusculaire sur des souris.

Les peptides selon l'invention présentent d'intéressantes propriétés vis-à-vis des entérotoxines ST naturelles humaines et animales.

Les essais effectués sur les peptides selon l'invention sont décrits ci-après notamment pour les peptides suivants:

- l'entérotoxine ST synthétique de porc conforme à l'invention, obtenue comme il a été décrit précédemment;
- le conjugué constitué par l'entérotoxine ST synthétique de porc et la toxine tétanique, ci-après désigné par STP-TT;
- le conjugué constitué par l'entérotoxine ST synthétique de porc et l'ovalbumine, ci-après désigné par STP-OVA.

Pour ces essais, on a également recours à l'entérotoxine ST naturelle humaine, purifiée commme indique ci-après.

## PURIFICATION D'ENTEROTOXINE ST NATURELLE HUMAINE

L'entérotoxine ST à purifier provient d'une souche humaine d'E. coli qui ne produit qu'une entérotoxine stable à la chaleur (souche fournie par le Dr Walter LAIRD de NIH - USA). Les conditions de croissance des bactéries et la purification de l'entérotoxine sont effectuées selon la méthode décrite par STAPLES et al. dans J. Biol. Chem. 255, 4716-4721.

Une seconde préparation d'entérotoxine ST pure d'origine humaine fournie par le Dr R. GIANNELLA est également utilisée dans l'étude présente.

## ETUDE DES PROPRIETES DES PEPTIDES SELON L'INVENTION

1° Non toxicité.

L'essai concernant la toxicité des peptides selon l'invention a été effectué sur des peptides obtenus selon la technique en phase solide décrite ci-dessus et dans laquelle les groupes SH des résidus de cystéine sont protégés par les acétamidométhyles.

La toxicité des peptides selon l'invention est étudiée à l'aide du test des souriceaux décrit dans J. Biol. Chem. 255, (1980), 4716-4721 par STAPLES et al. Ce test consiste à déterminer l'effet de l'administration, à des souriceaux, d'un composé, sur l'accumulation du fluide intestinal.

Pour ce faire, on injecte le composé à tester dans l'estomac de souriceaux ayant 3 ou 4 jours. On met ensuite

les souriceaux dans une étuve à 37° C pendant environ une heure puis on les anesthésie et on retire l'intestin. Si l'intestin est gonflé, cela résulte d'une accumulation de fluide dans l'intestin provoquée par le composé injecté qui est donc biologiquement actif et par conséquent toxique. De façon quantitative, on considère que le composé testé est biologiquement actif, lorsque le rapport entre le poids de l'intestin et le poids du corps est au moins égal à 0,08, étant indiqué que si on injecte de l'eau dans un essai témoin, le rapport poids de l'intestin/poids du corps est de 0,05. Dans les figures mentionnées ci-après, chaque point expérimental correspond à la moyenne des résultats obtenus avec trois souriceaux.

On a représenté, sur la figure 1, les résultats obtenus dans le cadre de l'étude de la toxicité de l'entérotoxine ST synthétique de porc. On a représenté en ordonnés, le rapport poids de l'intestin/poids du corps et chaque colonne représente la moyenne de trois déterminations. La colonne 4 correspond au résultat obtenu avec l'entérotoxine ST naturelle humaine administrée à raison de 9 ng. La colonne 5 correspond au résultat obtenu dans l'essai contrôle, sans entérotoxine. Les trois colonnes blanches désignées par 1, 2 et 3, correspondent aux résultats obtenus avec l'entérotoxine ST synthétique administrée à raison de 50 µg (colonne 1), 500 ng (colonne 2) et 50 ng (colonne 3).

De l'étude de ce graphe, on en déduit que l'entérotoxine ST synthétique de porc est incapable de provoquer l'accumulation de fluide dans le tractus intestinal des souris et par conséquent n'est pas toxique.

La non toxicité des peptides selon l'invention est en accord avec le fait qu'ils ne présentent pas de ponts disulfures intramoléculaires, étant donné que les groupes SH des résidus cystéyle sont protégés par des groupes protecteurs, stables en milieu biologique. Or, on a montré que les ponts disulfures intramoléculaires étaient indispensables à l'activité biologique de la toxine ST naturelle de porc et humaine (STAPLES et al. dans J. Biol. Chem. 255, 4716-4721 (1980).

2° Propriétés immunogènes.

Les peptides selon l'invention se révélant non toxiques, ils sont testés sur le plan immunologique.

Les essais ci-dessous indiqués effectués sur les conjugués selon l'invention permettent de montrer:

a) que les conjugués selon l'invention induisent des anticorps;

b) que ces anticorps réagissent de façon spécifique avec les peptides selon l'invention;

c) que ces anticorps reconnaissent l'entérotoxine ST naturelle (porcine ou humaine);

d) que ces anticorps neutralisent l'activité biologique de l'entérotoxine ST naturelle (porcine ou humaine).

Dans les essais dont il est question ciaprès, on utilise les deux conjugués suivants conformes à l'invention:

1° Conjugué constitué par l'entérotoxine ST synthétique de porc et l'ovalbumine (ci-après désigné par STP-OVA);

2° Conjugué constitué par l'entérotoxine ST synthétique de porc et la toxine tétanique (ci après désigné par STP-TT);

pour immuniser respectivement des lapins et des souris, selon le procédé préconisé par FRANTZ et ROBERTSON décrit dans Infect. and Immunity, 33, 193-198 (1981) en remplaçant la toxine naturelle par les deux conjugués.

a) Les conjugués selon l'invention induisent des anticorps.

a)1° Immunisation à l'aide du conjugué entérotoxine ST synthétique de porc-ovalbumine.

Les préparations du susdit conjugué selon l'invention contenant 100 µg d'entérotoxine ST synthétique de porc par ml sont mélangées avec une quantité égale d'adjuvants de FREUND.

On injecte par voie intradermique à des lapins BOUSCAT (6 mois), 1 ml de conjugué STP-OVA à des endroits multiples, le long du dos.

Des doses vaccinantes (50 µg d'entérotoxine ST synthétique de porc) en suspension dans l'adjuvant de FREUND incomplet sont effectuées à 4 semaines d'intervalle pendant 3 mois.

On recueille le sérum de lapin, trois semaines après la dernière injection et on le stocke à -20° C jusqu'à ce qu'il soit soumis à la méthode d'Elisa.

a)2° Immunisation à l'aide du conjugué entérotoxine ST synthétique de porc-toxine tétanique.

On immunise des souris (Balb/c) avec le conjugué STP-TT selon le protocole suivant. On mélange 100 µl de conjugué STP-TT (contenant 10 µg d'entérotoxine ST synthétique de porc) avec un volume égal d'adjuvant complet de FREUND. On injecte cette préparation de façon intrapéritonéale.

Des injections vaccinantes sont faites sous forme d'injections intramusculaires (100 µl de conjugué selon l'invention et 100 µl d'adjuvant incomplet de FREUND), chaque mois, pendant deux mois. On recueille le sérum de souris, 4 jours après la dernière injection. Le sérum est stocké à -20° C jusqu'à ce qu'il soit soumis à la méthode d'Elisa.

a)3° Détermination des anticorps par la méthode d'Elisa (Enzyme Linked Immunosorbent Assay).

La méthode d'Elisa est une technique en phase hétérogène (Enzyme Linked Immunosorbent Assay) qui permet de déterminer et de doser des anticorps au moyen d'une réaction antigène-anticorps.

De façon générale, on a recours à des tubes en plastique que l'on recouvre d'un excès d'antigènes, puis on introduit le sérum contenant les anticorps à déterminer et on laisse incuber.

Au cours de l'incubation, les anticorps s'associent avec leurs antigènes respectifs. On effectue ensuite une deuxième incubation à l'aide d'anticorps anti-immunoglobuline marqués grâce à une enzyme.

Plus précisément, l'essai concernant la détermination des anticorps contenus dans le sérum de lapin et de souris, respectivement immunisé avec les conjugués STP-OVA et STP-TT, comme indiqué ci-dessus, a été effectué comme il est décrit par VOLLER et al. dans Enzyme linked immunosorbent assay, a guide with abstracts of microplates applications, Dynatech, Europe, Guernsey, p. 1 (1979).

Les entérotoxines ST synthétiques de porc ou les entérotoxines ST naturelles de porc (20 µg/ml dans 50 mM de tampon carbonate/bicarbonate à pH 9,0) (tampon de revêtement) sont déposées en excès sur les tubes des microplaques Elisa (commercialisés par Nunc Inter-Med Denmark) pendant environ deux heures à environ 37°C.

Après dépôt, on lave les tubes à l'aide d'une solution saline de tampon phosphate de sodium à pH 7,4 contenant 0,05 % de Tween® 20 (agent mouillant obtenu par condensation des esters d'acides gras et de sorbitol avec l'oxyde d'éthylène, la fixation des chaînes polyoxyéthyléniques s'effectuant sur les hydroxyles non estérifiés du sorbitol et commercialisé par la société MERCK sous le nom de Tween® 2O)(PiNa/Tween® 20). Le même tampon (PiNa/Tween® 20) est également utilisé comme diluant des sérums de lapin et de souris à tester et comme diluant des anticorps anti-immunoglobulines marqués par l'enzyme.

Ces anticorps anti-immunoglobulines sont d'une part des IgG chèvre anti-lapin (produits par les laboratoires BIONETIC, USA), d'autre part des IgG mouton antisouris (INSTITUT PASTEUR, France), tous les deux couplés au même enzyme, qui est la peroxydase.

Les sérums de lapin et de souris à tester sont ajoutés dans les tubes dans des dilutions appropriées et on laisse incuber pendant une heure à 37°C. Après d'autres lavages avec PiNa/Tween® 20, on ajoute les anticorps anti-immunoglobulines respectifs ci-dessus définis et on laisse incuber pendant une heure à 37°C. Après plusieurs lavages, la quantité d'enzymes liées aux tubes est déterminée en utilisant 1'0-phénylène diamine (50 mg/100 ml) et 40 µ1 de peroxyde d'hydrogène dans 50 mM de tampon citrate/phosphate à pH 5, comme substrat. On arrête la réaction 30 minutes après, par addition de 50 µl de $H_2SO_4$ à 12,5 % et on lit immédiatement les valeurs d'absorption à 492 nm.

b)1° <u>Les anticorps induits par les conjugués STp-OVA et STP-TT reagissent avec l'enterotoxine synthétique de porc.</u>

On étudie dans le sérum de lapin à tester, la possibilité qu'il a de se combiner avec l'entérotoxine ST synthétique de porc.

La figure 2 représente la courbe de dosage (par la méthode Elisa) du sérum de lapin comportant des anticorps induits par le conjugué STP-OVA.

On a représenté en ordonnées la variation de densité optique en fonction de l'inverse des dilutions de sérum.

La ligne en points noirs concerne le dosage du sérum contenant les anticorps induits par le conjugué STP-OVA.

La ligne enpoints blancs concerne le sérum contenant les anticorps induits par le conjugué STP-OVA, préincubé pendant une heure, à la température de la pièce avec un excès d'entérotoxine ST synthétique de porc, avant la méthode Elisa.

Comme il résulte clairement de cette figure lorsqu'un excès d'entérotoxine ST synthétique de porc est incubée avec le sérum avant l'addition à l'entérotoxine ST synthétique liée aux parois plastiques du tube, on n'observe aucune réaction, ce qui indique que la liaison entre les anticorps induits par le conjugué STP-OVA et l'entérotoxine ST synthétique de porc est spécifique.

b)2° <u>Les anticorps induits par les conjugués STP-OVA et STP-TT réagissent avec l'entérotoxine ST synthétique humaine.</u>

Lorsque le sérum de lapin contenant les anticorps induits par le conjugué STP-OVA est testé sur la toxine ST synthétique humaine, on observe une réaction croisée.

La figure 3 représente la courbe de dosage (par la méthode Elisa) du sérum de lapin contenant les anticorps induits par le conjugué STP-OVA par l'entérotoxine ST synthétique humaine.

On a représenté en ordonnées la variation de densité optique et en abscisses l'inverse des dilutions de sérum.

La courbe portant les triangles correspond aux résultats obtenus avec le sérum comportant les anticorps induits par le conjugué STP-OVA.

La courbe en points blancs correspond aux résultats obtenus avec le sérum contenant les anticorps induits par le conjugué STP-OVA, préincubé pendant environ une heure, à la température ambiante avec un excès d'entérotoxine ST synthétique humaine, avant d'effectuer la méthode Elisa.

Il résulte clairement de cette figure que les anticorps induits par le conjugué STP-OVA, réagissent avec l'entérotoxine ST synthétique humaine qui est fixée sur les tubes.

On remarque donc à nouveau que l'entéroxine ST synthétique humaine en excès, incubée avec le sérum à tester, avant d'effectuer le test Elisa, bloque la liaison spécifique des anticorps induits par le conjuqué STP-OVA.

c) <u>Les anticorps induits par le conjugué STP-OVA reconnaissent l'entérotoxine ST naturelle humaine.</u>

Afin de montrer que les anticorps fabriqués contre l'entérotoxine ST synthétique de porc sont capables de réagir avec l'entérotoxine ST naturelle humaine, on dépose dans des cupules d'une microplaque Elisa de l'entérotoxine ST naturelle humaine et de l'entérotoxine ST synthétique humaine.

On effectue le test Elisa dans ces conditions, et on observe que les susdits anticorps réagissent avec l'entérotoxine ST naturelle humaine.

On peut montrer de la même façon que les anticorps fabriqués contre l'entérotoxine ST synthétique de porc reconnaissent l'entérotoxine ST naturelle de porc.

On en déduit donc que les -peptides selon l'invention induisent des anticorps qui reconnaissent:

- l'entérotoxine ST synthétique de porc,
- l'entérotoxine ST synthétique humaine,

- l'entérotoxine ST naturelle de porc,
- l'entérotoxine ST naturelle humaine.

d) Les peptides selon l'invention induisent des anticorps qui neutralisent l'entérotoxine ST naturelle humaine.

On teste les sérums de lapins et de souris qui ont été respectivement immunisés avec les conjugués STP-OVA et STP-TT, pour déterminer leur capacité à neutraliser l'activité biologique de l'entérotoxine ST naturelle humaine en ayant recours au test des souriceaux décrit dans J. Biol. Chem. 255 (1980), 4716-4721 par STAPLES et al.

Pour chaque essai, on effectue les six dilutions suivantes de sérum dans un tampon PiNa, pH 7,00:

1° 1/50 2° 1/200 3° 1/400
4° 1/800 5° 1/1600 6° 1/3200

et on mélange le sérum à tester avec l'entérotoxine ST naturelle humaine.

d) 1° Résultats obtenus avec les anticorps induits par le conjugué STP-OVA.

Sur la figure 4, on a représenté l'effet du sérum de lapin comportant les anticorps induits par le conjugué STP-OVA sur l'accumulation de fluide intestinal provoqué par 12,5 ng d'entérotoxine ST naturelle humaine (12,5 ng d'entérotoxine ST représentant 5 unités de souris, cf. GIANNELLA R. A. (1976) Infect. and Immunity, 14, 95-99).

On a porté en ordonnées le rapport poids de l'intestin/poids du corps, et en abscisses les six dilutions de sérum.

Pour chaque dilution de sérum, on mélange 12,5 ng d'entérotoxine ST naturelle humaine avec 20 µl de sérum (volume total 100 µl) et on laisse incuber pendant une heure avant d'effectuer le test des souriceaux.

Chacun des points de la courbe obtenue correspond à la moyenne de trois déterminations.

La colonne 1 sert de témoin et correspond aux résultats obtenus avec 12,5 ng d'entérotoxine ST naturelle humaine.

La colonne 2 sert de témoin et correspond aux résultats obtenus avec 12,5 ng d'entérotoxine ST naturelle humaine à laquelle on a additionné 20 µl de sérum de lapin non immunisé dilué à 1/50.

La colonne 3 sert de témoin et correspond aux résultats obtenus sans entérotoxine.

d) 2° Résultats obtenus avec les anticorps induits par le conjugué STP-TT.

La figure 5 représente l'effet du sérum de souris comportant les anticorps induits par le conjugué STP-TT sur l'accumulation du fluide intestinal provoqué par 12,5 ng d'entérotoxine ST naturelle humaine.

La courbe de la figure 5 est établie comme il a été indiqué à propos de la figure 4 et les colonnes 1, 2 et 3 ont les significations indiquées à propos de la figure 4.

Les anticorps induits par les conjugués selon l'invention présentent la remarquable propriété de neutraliser, de façon spécifique, l'activité biologique de l'entérotoxine ST naturelle humaine.

Les peptides selon l'invention qui peuvent être obtenus par le procédé de synthèse en phase solide présentent l'avantage d'être préparés en grandes quantités et sous forme de matériaux homogènes, ce qui évite d'avoir recours aux méthodes compliquées consistant à faire croître des bactéries, suivie d'un procédé de purification astreignant.

Les peptides selon l'invention ne sont pas toxiques, même en grande quantité comme les tests l'ont prouvé. Ceci peut être expliqué par le fait qu'il n'y a pas de ponts disulfures, puisque les groupes SH des résidus cystéyle sont protégés par des groupements stables dans un milieu biologique.

Les peptides selon l'invention présentent l'intéressante propriété, lorsqu'ils sont couplés à différentes protéines porteuses, d'induire des anticorps qui reconnaissent l'entérotoxine ST naturelle, qui établissent des liaisons avec cette entérotoxine et qui neutralisent son activité biologique.

Ce résultat pourrait être expliqué par l'hypothèse que les quatre premiers résidus Asn-Thr-Phe-Tyr de l'extrémité N-terminale de la séquence des 18 aminoacides de l'entérotoxine ST ne sont probablement pas impliqués dans la configuration repliée induite par les ponts disulfures.

Cette portion de l'entérotoxine ST paraît consister en un déterminant antigénique stable, qui est moins immunogène que dans l'entérotoxine ST naturelle repliée, mais qui provoque des anticorps lorsqu'il fait partie de l'entérotoxine ST naturelle dont les groupes thiols des résidus cystéyle ont été réduits ou lorsqu'il se trouve dans la séquence peptidique des peptides selon l'invention et notamment de l'entérotoxine ST synthétique, dont les groupes thiols des résidus cystéyle sont protégés.

Les anticorps qui sont dirigés vers cette région de la molécule se lient à l'entérotoxine ST naturelle quelle que soit sa configuration.

L'exemple décrit ci-dessous montre la mise en évidence de la toxine ST dans un système de culture.

**Exemple de mise en évidence de la toxine ST dans un système de culture.**

Les bactéries isolées du prélèvement sont cultivées pendant 12 heures dans un milieu nutritif liquide, par exemple un milieu extrait de levure, hydrolysat de aséine (désigné par CYE).

Les bactéries, après culture, sont centrifugées et le surnageant de la centrifugation est récupéré.

On dose par procédé radio-immunologique classique la toxine contenue dans le surnageant. Ainsi les anticorps induits par les peptides selon l'invention permettent de doser la toxine ST.

Les peptides selon l'invention, seuls ou associés avec une molécule porteuse, sont susceptibles d'induire,

comme cela a été montré ci-dessus la synthèse d'anticorps neutralisants, ce qui permet d'utiliser les peptides comme agents vaccinants.

A titre d'exemple, les peptides selon l'invention (en association avec une molécule porteuse telle que la toxine tétanique) sont utilisés à raison d'environ:

- 10 µg pour des souris (souris Balb/c d'environ 20 g);
- 50 µg pour des lapins de type Bouscat.

L'invention vise également l'application des peptides à la mise au doint des tests radio-immunologiques et immuno-enzymatiques (ELISA) pour détecter directement l'entérotoxine ST ainsi qu'à l'immunisation dans le domaine médical et vétérinaire contre l'entérotoxine ST naturelle.

L'invention ne se limite naturellement pas aux peptides particuliers qui ont été envisagés.

Comme il est bien connu de l'homme de l'art, certains résidus amino-acyle contenus dans les séquences P peuvent éventuellement être remplacés par d'autres résidus amino-acyle, dans la mesure où ceux-ci ne modifient pas sensiblement les configurations de surface des peptides formés ou la capacité des anticorps induits par les peptides ainsi modifiés à l'égard des peptides non modifiés ou dans le cas présent, contre les enterotoxines ST naturelles. A cet égard, on mentionnera par exemple les éventuelles substitutions du groupe alanyle par le groupe glycyl ou vice-versa, la substitution possible des résidus iso-asparagiques par des résidus aspartiques, glutamine ou isoglutamine, la substitution des groupes valine par les groupes alanine, leucine ou glycine, la substitution des groupes lysine par des groupes norleucine ou encore arginine, etc., sous réserve que soit chaque fois vérifiée la capacité des peptides modifiés d'induire des anticorps susceptibles de neutraliser les peptides tels qu'ils ont été définis plus haut, ou la toxine naturelle. Il est naturellement entendu que tous ces équivalents possibles sont couverts par les peptides plus spécifiquement revendiqués ci-après.

## Revendications

1. Peptide P-(P')n comportant au plus 18 (n+1) amino-acides et au moins 4 (n+1) amino-acides, lévogyres, caractérisé en ce que:

- n vaut 0 ou 1

et en ce que lorsque n vaut 0, la séquence peptidique P est la séquence suivante (3):

Asn-Thr-Phe-Tyr-Cys-Gly-Gly-Gly

ou est contenue dans l'enchaînement peptidique suivant:

Asn-Thr-Phe-Tyr-Cys-Cys-Glu-Leu-Cys-Cys-A-Pro-Ala-Cys-Ala-Gly-Cys-T(I)
(N-ter) (C-ter)

dans laquelle soit A représente Asn et T représente Tyr, soit A représente Tyr et T représente Asn;

et caractérisé en ce que les groupes thiols des éventuels résidus cystéyle présents dans la molécule ont été protégés par des groupements stables dans les conditions biologiques, l'un au plus des groupes thiols pouvant être sous forme de groupe SH libre ou pouvant être protégé par un groupe non stable dans les conditions biologiques; et en ce que lorsque n vaut 1, la séquence peptidique P-P' est constituée par deux séquences peptidiques P et P' identiques ou différentes comportant chacune au plus 18 acides aminés et au moins 4 acides amines, contenues dans l'enchaînement peptidique de formule (I) ci-dessus indiquée, les deux séquences peptidiques P et P' étant reliées entre elles:

- par l'intermédiaire d'une liaison disulfure établie entre l'atome de soufre de l'un quelconque des résidus cystéyle de l'une des deux séquences et l'atome de soufre de l'un quelconque des résidus cystéyle de l'autre séquence;
- ou per l'intermédiaire d'une liaison établie entre le groupe carboxyle de l'une des deux séquences peptidiques P et P' et le groupe aminé de l'autre séquence;

et caractérisé en ce que l'un au plus des groupes thiols, s'il n'est pas engagé dans une liaison disulfure, peut être sous forme de groupe SH libre, ou protégé par un groupe non stable dans les conditions biologiques et les autres groupes thiols des éventuels résidus cystéyle sont protégés par un groupement protecteur stable dans les conditions biologiques.

2. Peptide P-(P')n selon la revendication 1, comportant au plus 18 (n+1) amino-acides et au moins 4 (n+1) amino-acides, lévogyres, caractérisé en ce que:

- n vaut 0 lorsque la séquence peptidique P ne comporte aucun résidu cystéyle;
- n vaut 0 ou 1, lorsque la séquence peptidique P comporte au moins un résidu cystéyle;

et en ce que lorsque n vaut 0, la séquence peptidique P est la séquence suivante (3):

Asn-Thr-Phe-Tyr-Cys-Gly-Gly-Gly

ou est contenue dans l'enchaînement peptidique suivant:

Asn-Thr-Phe-Tyr-Cys-Cys-Glu-Leu-Cys-Cys-A-Pro-Ala-Cys-Ala-Gly-Cys-T(I)
(N-ter) (C-ter)

dans laquelle soit A représente Asn et T représente Tyr, soit A représente Tyr et T représente Asn;

et caractérisé en ce que les groupes thiols des éventuels résidus cystéyle présents dans la molécule ont été protégés par des groupements stables dans les conditions biologiques, l'un au plus des groupes thiols pouvant être sous forme de groupe SH libre ou pouvant être protégé par un groupe non stable dans les conditions biologiques;

et en ce que lorsque n vaut 1 la séquence peptidique P-P' est constituée par deux séquences peptidiques P et P' identiques comportant chacune au plus 18 acides aminés et au moins 4 acides aminés, contenues dans l'enchaînement peptidique de formule (I) ci-dessus indiquée, les deux séquences peptidiques P et P' étant reliées entre elles:

- par l'intermédiaire d'une liaison disulfure établie entre l'atome de soufre de l'un quelconque des résidus cystéyle de l'une des séquences et l'atome de soufre de l'un quelconque des résidus cystéyle de l'autre séquence,

et caractérisé en ce que les autres groupes thiols non engagés dans la liaison disulfure des éventuels résidus cystéyle sont protégés par un groupement protecteur stable dans les conditions biologiques.

3. Peptide selon la revendication 1 ou 2, caractérisé en ce que le groupe protecteur stable dans les conditions biologiques de la fonction thiol des résidus cystéyle a pour formule:

$$R\underset{\underset{O}{\|}}{C}NR'R''$$

dans laquelle R, R' et R'' représentent indépendamment les uns des autres un atome d'hydrogène, un radical alcoyle de 1 à 4 atomes de carbone, de préference:

$$CH_3\underset{\underset{O}{\|}}{C}NHR' \qquad R\underset{\underset{O}{\|}}{C}NHCH_3 \qquad \text{et} \qquad R-\underset{\underset{O}{\|}}{C}-\underset{\underset{R'}{|}}{N}-\underset{\underset{R''}{|}}{CH_2}$$

dans lesquels R et R' représentent un atome d'hydrogène, un radical alcoyle ayant de 1 à 4 atomes de carbone.

4. Peptide selon l'une quelconque des revendications 1 et 3, caractérisé en ce que le groupe protecteur de la fonction thiol est constitué par l'acétamidométhyle ou le formamidométhyle.

5. Peptides monomères selon l'une quelconque des revendications 1 à 4, caractérisés en ce que leur enchaînement peptidique est la séquence suivante (3): Asn-Thr-Phe-Tyr-Cys-Gly-Gly-Gly ou est contenu dans la séquence peptidique suivante:

Asn-Thr-Phe-Tyr-Cys-Cys-Glu-Leu-Cys-Cys-A-Pro-Ala-Cys-Ala-Gly-Cys-T

(N-ter) (C-ter)

dans laquelle soit A représente Asn et T représente Tyr, soit A représente Tyr et T représente Asn; et caractérisés en ce que les groupes thiols des éventuels résidus cystéyle présents dans la molécule ont été protégés par des groupements stables dans les conditions biologiques, l'un au plus des groupes thiols pouvant être sous forme de groupe SH libre ou pouvant être protégé par un groupe non stable dans les conditions biologiques.

6. Peptides monomères selon la revendication 5, caractérisés en ce que tous les groupes thiols des éventuels résidus cystéyle ont été protégés par des groupements stables dans les conditions biologiques.

7. Peptides monomères selon la revendication 5, caractérisés en ce que tous les groupes thiols des éventuels résidus cystéyle, sauf un, ont été protégés par des groupements stables dans les conditions biologiques.

8. Peptides dimères selon l'une quelconque des revendications 1 à 4, caractérisés en ce qu'ils sont constitués par deux séquences peptidiques P et P' identiques comportant chacune au plus 18 acides aminés et au moins 4 acides aminés, contenues dans l'enchaînement peptidique de formule (I) ci-dessus indiquée, les deux séquences peptidiques P étant reliées entre elles par l'intermédiaire d'une liaison disulfure établie entre l'atome de soufre de l'un des résidus cystéyle de l'une des deux séquences peptidiques et l'atome de soufre de l'un des résidus cystéyle de l'autre séquence peptidique, et caractérisés en ce que les autres groupes thiols -non engagés dans la liaison disulfure- des éventuels résidus cystéyle sont protégés par un groupement protecteur stable dans les conditions biologiques.

9. Peptides selon l'une quelconque des revendications 1 à 8, caractérisés en ce qu'ils comprennent l'une des séquences suivantes:

-Asn-Thr-Phe-Tyr

-Asn-Thr-Phe-Tyr-Cys

-Cys-Cys-Asn-Pro-Ala-Cys

-Cys-Cys-Tyr-Pro-Ala-Cys

-Asn-Thr-Phe-Tyr-Cys-Cys-Glu

-Asn-Thr-Phe-Tyr-Cys-Cys-Glu-Leu

-Cys-Cys-Gly-Leu-Cys-Cys-Asn-Pro-Ala-Cys-Ala-Gly-Cys
-Cys-Cys-Gly-Leu-Cys-Cys-Tyr-Pro-Ala-Cys-Ala-Gly-Cys

10. Peptides monomères selon l'une quelconque des revendications 5 à 7, caractérisés en ce qu'ils répondent à la formule suivante:

Asn-Thr-Phe-Tyr
Asn-Thr-Phe-Tyr-Cys
Cys-Cys-Asn-Pro-Ala-Cys
Cys-Cys-Tyr-Pro-Ala-Cys
Asn-Thr-Phe-Tyr-Cys-Cys-Glu
Asn-Thr-Phe-Tyr-Cys-Cys-Glu-Leu
Cys-Cys-Gly-Leu-Cys-Cys-Asn-Pro-Ala-Cys-Ala-Gly-Cys
Cys-Cys-Gly-Leu-Cys-Cys-Tyr-Pro-Ala-Cys-Ala-Gly-Cys
Asn-Thr-Phe-Tyr-Cys-Cys-Gly-Leu-Cys-Cys-Asn-Pro-Ala
Cys-Ala-Gly-Cys-Tyr (1)
Asn-Thr-Phe-Tyr-Cys-Cys-Gly-Leu-Cys-Cys-Tyr-Pro-Ala
Cys-Ala-Gly-Cys-Asn (2)
Asn-Thr-Phe-Tyr-Cys-Gly-Gly-Gly (3)

11. Conjugués caractérisés en ce qu'ils sontconstitués par un peptide selon l'une quelconque des revendications 1 à 10, en association avec une molécule porteuse physiologiquement acceptable et non toxique, de préférence une protéine avantageusement choisie parmi l'ovalbumine, la toxine tétanique, le choléragénoïde et la cytotoxine de Shigella.

12. Application d'un peptide selon l'une quelconque des revendications 1 à 11, à la fabrication d'anticorps permettant la mise en évidence des toxines ST humaines ou animales, notamment porcines.

13. Application d'un peptide selon l'une quelconque des revendications 1 à 11, à la fabrication d'anticorps permettant la vaccination de l'homme et des animaux vis-à-vis des entérotoxines ST humaines ou animales notamment porcines.

## Patentansprüche

1. Peptid P-$(P')_n$ bestehend aus höchstens 18 (n+1) linksdrehenden Aminosäuren und mindestens 4 (n+1) linksdrehenden Aminosäuren, dadurch gekennzeichnet, daß n gleich 0 oder 1 ist, und daß, wenn n gleich 0 ist, die Peptidsequenz P die folgende Sequenz (3) ist:

Asn-Thr-Phe-Tyr-Cys-Gly-Gly-Gly

oder in der folgenden Peptidkette enthalten ist:

Asn-Thr-Phe-Tyr-Cys-Cys-Glu-Leu-Cys-Cys-A-Pro-Ala-Cys-Ala-Gly-Cys-T (I)
(N-Ende) (C-Ende)

worin entweder A Asn und T Tyr oder A Tyr und T Asn bedeutet; und gekennzeichnet dadurch, daß die Thiolgruppen der möglichen Cysteylreste, die in dem Molekül vorhanden sind, geschützt worden sind durch Gruppen, die unter biologischen Bedingungen stabil sind, wobei höchstens eine der Thiolgruppen in Form einer freien SH-Gruppe oder geschützt durch eine Gruppe, die unter biologischen Bedingungen nicht stabil ist, sein darf, und daß, wenn n gleich 1 ist, die Peptidsequenz P-P' gebildet wird aus zwei Peptidsequenzen P und P', die identisch oder verschieden sind und wobei jede höchstens 18 Aminosäuren und mindestens 4 Aminosäuren enthält, die in der Peptidkette, die als Formel (I) oben angegeben ist, enthalten sind, wobei die beiden Peptidsequenzen P und P' miteinander verbunden sind durch eine Disulfidbindung, die zwischen dem Schwefelatom irgendeines Cysteylrestes einer der beiden Sequenzen und dem Schwefelatom irgendeines Cysteylrestes der anderen Sequenz gebildet wird; oder durch eine Bindung, die zwischen der Carboxylgruppe einer der beiden Peptidsequenzen P und P' und der Aminogruppe der anderen Sequenz gebildet wird; und gekennzeichnet dadurch, daß höchstens eine der Thiolgruppen, wenn sie nicht in der Disulfidbindung gebunden ist, in Form einer freien SH-Gruppe oder geschützt durch eine Gruppe, die unter biologischen Bedingungen nicht stabil ist, vorliegen kann und daß die anderen Thiolgruppen der möglichen Cysteylreste geschützt sind durch eine Schutzgruppe, die unter biologischen Bedingungen stabil ist.

2. Peptid P-$(P')_n$ nach Anspruch 1, enthaltend höchstens 18 (n+1) linksdrehende Aminosäuren und mindestens 4 (n+1) linksdrehende Aminosäuren, dadurch gekennzeichnet, daß n gleich 0 ist, wenn die Peptidsequenz P keinen Cysteylrest enthält;n gleich 0 oder 1 ist, wenn die Peptidsequenz P mindestens einen Cysteylrest enthält; und daß, wenn n gleich 0 ist, die Peptidsequenz P die folgende Sequenz (3) ist:

Asn-Thr-Phe-Tyr-Cys-Gly-Gly-Gly

oder in der folgenden Peptidkette enthalten ist:

Asn-Thr-Phe-Tyr-Cys-Cys-Glu-Leu-Cys-A-Pro-Ala-Cys-Ala-Gly-Cys-T (I)
(N-Ende) (C-Ende)

worin entweder A Asn und T Tyr oder A Tyr und T Asn bedeutet; und gekennzeichnet dadurch, daß die Thiolgruppen der möglichen Cysteylreste, die in dem Molekül vorhanden sind, geschützt worden sind durch unter biologischen Bedingungen stabile Gruppen, wobei höchstens eine der Thiolgruppen in der Form einer freien SH-Gruppe oder geschützt durch eine unter biologischen Bedingungen nicht stabile Gruppe vorliegen

kann; und daß, wenn n gleich 1 ist, die Peptidsequenz P-P' durch zwei Peptidsequenzen P und P' gebildet wird, die identisch sind und höchstens 18 Aminosäuren und mindestens 4 Aminosäuren enthalten, die in der Peptidkette der Formel (I), die oben angegeben ist, enthalten sind, und wobei die zwei Peptidsequenzen P und P' miteinander verbunden sind durch eine Disulfidbindung, die zwischen dem Schwefelatom eines der Cysteylreste von einer der beiden Sequenzen und dem Schwefelatom eines der Cysteylreste der anderen Sequenz gebildet wird; und gekennzeichnet dadurch, daß die anderen Thiolgruppen der möglichen Cysteylreste, die nicht in der Disulfidbindung gebunden sind, geschützt sind durch eine Schutzgruppe, die unter biologischen Bedingungen stabil ist.

3. Peptid nach Anspruch 1 oder 2, dadurch gekennzeichnet, daß die unter biologischen Bedingungen stabile Schutzgruppe der Thiolfunktion der Cysteylreste die folgende Formel hat:

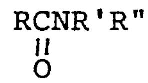

worin R, R' und R'' unabhängig voneinander Wasserstoff, einen Alkylrest mit 1 bis 4 Kohlenstoffatomen, vorzugsweise:

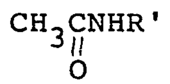

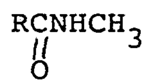

und

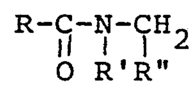

bedeuten, wobei R und R' Wasserstoff, einen Alkylrest mit 1 bis 4 Kohlenstoffatomen bedeuten.

4. Peptid nach einem der Ansprüche 1 bis 3, dadurch gekennzeichnet, daß die Schutzgruppe der Thiolfunktion von einer Acetamidomethylgruppe oder Formamidomethylgruppe gebildet wird.

5. Monomere Peptide nach einem der Ansprüche 1 bis 4, dadurch gekennzeichnet, daß ihre Peptidkette die folgende Sequenz (3)

Asn-Thr-Phe-Tyr-Cys-Gly-Gly-Gly

ist oder in der folgenden Peptidsequenz:

Asn-Thr-Phe-Tyr-Cys-Cys-Glu-Leu-Cys-Cys-A-Pro-Ala-Cys-Ala-Cly-Cys-T

(N-Ende) (C-Ende)

enthalten ist und worin A Asn und T Tyr oder A Tyr und T Asn bedeutet; und gekennzeichnet dadurch, daß die Thiolgruppen der möglichen Cysteylreste, die in dem Molekül vorhanden sind, geschützt worden sind mit unter biologischen Bedingungen stabilen Gruppen, und wobei höchstens eine der Thiolgruppen in Form einer freien SH-Gruppe vorliegen darf oder geschützt durch eine Gruppe, die unter biologischen Bedingungen nicht stabil ist.

6. Monomere Peptide nach Anspruch 5, dadurch gekennzeichnet, daß alle Thiolgruppen der möglichen Cysteylreste durch Gruppen, die unter biologischen Bedingungen stabil sind, geschützt worden sind.

7. Monomere Peptide nach Anspruch 5, dadurch gekennzeichnet, daß alle Thiolgruppen der möglichen Cysteylreste, mit Ausnahme von einer, durch Gruppen, die unter biologischen Bedingungen stabil sind, geschützt worden sind.

8. Dimere Peptide nach einem der Ansprüche 1 bis 4, dadurch gekennzeichnet, daß sie durch zwei identische Peptidsequenzen P und P' gebildet werden, wobei jede höchstens 18 Aminosäuren und mindestens 4 Aminosäuren beinhaltet, die in der Peptidkette der Formel (I), wie oben angegeben, enthalten sind, wobei die beiden Peptidsequenzen P miteinander verbunden sind durch eine Disulfidbindung, die zwischen dem Schwefelatom des einen der Cysteylreste der einen der beiden Peptidsequenzen und dem Schwefelatom des einen der Cysteylreste der anderen Peptidsequenz gebildet wurde; und dadurch gekennzeichnet, daß die anderen Thiolgruppen der möglichen Cysteylreste, die die Disulfidbindung nicht bilden, durch eine Schutzgruppe, die stabil ist unter biologischen Bedingungen, geschützt werden.

9. Peptide nach einem der Ansprüche 1 bis 8, dadurch gekennzeichnet, daß sie eine der folgenden Sequenzen enthalten:

-Asn-Thr-Phe-Tyr
-Asn-Thr-Phe-Tyr-Cys
-Cys-Cys-Asn-Pro-Ala-Cys
-Cys-Cys-Tyr-Pro-Ala-Cys
-Asn-Thr-Phe-Tyr-Cys-Cys-Glu
-Asn-Thr-Phe-Tyr-Cys-Cys-Glu-Leu

-Cys-Cys-Gly-Leu-Cys-Cys-Asn-Pro-Ala-Cys-Ala-Gly-Cys
-Cys-Cys-Gly-Leu-Cys-Cys-Tyr-Pro-Ala-Cys-Ala-Gly-Cys-.

10. Monomere Peptide nach einem der Ansprüche 5 bis 7,-dadurch gekennzeichnet, daß sie einer der folgenden Formeln entsprechen:
Asn-Thr-Phe-Tyr
Asn-Thr-Phe-Tyr-Cys
Cys-Cys-Asn-Pro-Ala-Cys
Cys-Cys-Tyr-Pro-Ala-Cys
Asn-Thr-Phe-Tyr-Cys-Cys-Glu
Asn-Thr-Phe-Tyr-Cys-Cys-Glu-Leu
Cys-Cys-Gly-Leu-Cys-Cys-Asn-Pro-Ala-Cys-Ala-Gly-Cys
Cys-Cys-Gly-Leu-Cys-Cys-Tyr-Pro-Ala-Cys-Ala-Gly-Cys
Asn-Thr-Phe-Tyr-Cys-Cys-Gly-Leu-Cys-Cys-Asn-Pro-Ala-Cys
Ala-Gly-Cys-Tyr (1)
Asn-Thr-Phe-Tyr-Cys-Cys-Gly-Leu-Cys-Cys-Tyr-Pro-Ala-Cys
Ala-Gly-Cys-Asn (2)
Asn-Thr-Phe-Tyr-Cys-Gly-Gly-Gly (3).

11. Konjugate, dadurch gekennzeichnet, daß sie gebildetwerden von einem Peptid nach einem der Ansprüche 1 bis 10 in Verbindung mit einem physiologisch annehmbaren und nicht toxischen Trägermolekül, vorzugsweise einem Protein, das vorteilhaft ausgewählt ist aus Ovalbumin, Tetanustoxin, Choleragenoid und Shigella-Cytotoxin.

12. Verwendung eines Peptids nach einem der Ansprüche 1 bis 11 zur Herstellung von Antikörpern, die den Nachweis von menschlichen oder tierischen ST-Toxinen, insbesondere bei Schweinen, ermöglichen.

13. Verwendung eines Peptids nach einem der Ansprüche 1 bis 11 zur Herstellung von Antikörpern, die die Impfung von Menschen oder Tieren gegen menschliche oder tierische ST-Enterotoxine, insbesondere von Schweinen, ermöglichen.

## Claims

1. Peptide P-(P')n comprising at the most 18 (n+1) levorotatory amino-acids and at least 4 (n+I) levorotatory amino-acids, characterized in that
- n is equal to 0 or 1,
and in that when n is equal to 0, the peptidic sequence P is the following sequence (3)
Asn-Thr-Phe-Tyr-Cys-Gly-Gly-Gly
or is contained in the following peptidic chain:
Asn-Thr-Phe-Tyr-Cys-Cys-Glu-Leu-Cys-Cys-A-Pro-Ala-Cys-Ala-Gly-Cys-T (I),
(N-ter) (G-ter)
in which either A represents Asn and T represents Tyr or A represents Tyr and T represents Asn;
and characterized in that the thiol groups of the possible cysteyl residues present in the molecule have been protected by groups which are stable under biological conditions, one at the most of the thiol groups being liable to be in the form of a free SH group or to be protected by a group which is not stable under biological conditions; and in that, when n is equal to 1, the peptidic sequence P-P' is constituted by two peptidic sequences P and P', identical or different each comprising, at the most 18 amino-acids and at least 4 amino-acids, contained in the peptidic chain of formula (I) above indicated, the two peptidic sequences P and P' being connected together
- through a disulfide linkage established between the sulfur atom of anyone of the cysteyl residues of one of the two sequences and the sulfur atom of anyone of the cysteyl residues of the other sequence;
- through a linkage established between the carboxyl group of one of the two peptidic sequences P and P' and the amino group of the other sequence;
and characterized in that one at most of the thiol groups, if it is not engaged in a disulfide linkage, can be in the form of a free SH group or protected by group which is not stable under biological conditions and the other thiol groups of the possible cysteyl residues are protected by a protective group which is stable under biological conditions,

2. Peptide P-(P')n according to claim 1, comprising at the most 18 (n+1) amino-acids and at least 4 (n+1) levorotatory amino-acids, characterized in that
- n is equal to 0 when the peptidic sequence does not include any cysteyl residue;
- n is equal to 0 or 1 when the peptidic sequence p includes at least one cysteyl residue
and in that when n is equal to 0, the peptidic sequence P is the following sequence (3)
Asn,Thr-Phe-Tyr-Cys-Gly-Gly-Gly
or is contained in the following peptidic chain
Asn-Thr-Phe-Tyr-Cys-Cys-Glu-Leu-Cys-Cys-A-Pro-Ala-Cys-Ala-Gly-Cys-T (I)
(N-ter) (C-ter)
in which either A represents Asn and T represents Tyr or A represents Tyr and T represents Asn;

and characterized in that the thiol groups of the possible cysteyl residues present in the molecule have been protected by stable groups under biological conditions, one at the most of the thiol groups being liable to be in the form of a free SH group or to be protected by a group non stable under biological conditions; and in that, when n is equal to 1, the peptidic sequence P-P′ is constituted by two peptidic sequences P and P′, identical or different comprising each, at the most 18 amino-acids and at least 4 aminoacids, contained in the peptidic chain of formula (I) above indicated, the two peptidic sequences P and P′ being connected together:

- through a disulfide linkage established between the sulfur atom of anyone of the cysteyl residues of one of the two sequences and the sulfur atom of anyone of the cysteyl residues of the other sequence:

and characterized in that the other thiol groups not engaged in the disulfide linkage of the possible cysteyl residues are protected by a protective group stable under biological conditions.

3. Peptide according to claim 1 or 2, characterized in that the the protective group stable under biological conditions of the thiol function of the cysteyl residues has the following formula

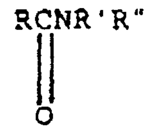

in which R, R′ and R′ represent independently of one another a hydrogen atom, an alkyl radical of 1 to 4 carbon atoms, preferably

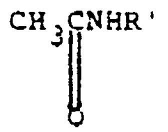 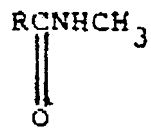 and 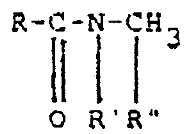

in which R and R′ represent a hydrogen atom, an alkyl radical from 1 to 4 carbon atomes.

4. Peptide according to anyone of claims from 1 to 3, characterized in that the protective group of the thiol function is constituted by acetamidomethyl or formamidomethyl.

5. Monomeric peptides according to anyone of claims from 1 to 4, characterized in that their peptidic chain is the following sequence

Asn-Thr-Phe-Tyr-Cys-Gly-Gly-Gly

is contained in the following sequence

Asn-Thr-Phe-Tyr-Cys-Cys-Glu-Leu-Cys-Cys-A-Pro-Ala-Cy-Ala-Gly-Cly-Cys-T(I)

(N-ter) (C-ter)

in which either A represents Asn and T represents Tyr or A represents Tyr and T represents Asn; and characterized in that the thiol groups of the possible cysteyl residues present in the molecule have been protected by stable groups under biological conditions, one at the most of the thiol groups being liable to be in the form of a free SH group or to be protected by a group non stable under biological conditions.

6. Monomeric peptides according to claim 5, characterized in that all the thiol groups of the possible cysteyl residues have been protected by groups, which are stable under biological conditions.

7. Monomeric peptides according to claim 5, characterized in that all the thiol groups of the possible cysteyl residues, except one, have been protected by groups which are stable under biological conditions.

8. Dimeric peptides according to anyone of claims from 1 to 4, characterized in that the they are constituted by two identical peptidic sequences P and P′, each including at the most 18 amino-acids and at least 4 amino-acids, contained in the peptidic chain of formula (I) as indicated above, the two peptidic sequences p being connected together through a disulfide linkage established between the sulfur atom of one of the cysteyl residues of the other peptidic sequence, and characterized in that the other thiol groups not engaged in the disulfide linkage of the possible cysteyl residues are protected by a protective group which are stable under biological conditions.

3. Peptides according to anyone of claims from 1 to 8, characterized in that they comprise one of the following sequences:

-Asn-Thr-Phe-Tyr
-Asn-Thr-Phe-Tyr-Cys
-Cys-Cys-Asn-Pro-Ala-Cys
-Cys-Cys-Tyr-Pro-Ala-Cys
-Asn-Thr-Phe-Tyr-Cys-Gys-Glu
-Asn-Thr-phe-Tyr-Cys-Cys-Glu-Leu
-Cys-Cys′Gly-Leu,Cys-Cys-Asn-Pro-Ala-Cys-Ala-Gly-Cys

-Cys-Cys-Gly-Leu-Cys-Cys-Tyr-Pro-Ala-Cys-Ala-Gly-Cys

10. Monomeric peptides according to anyone ofclaims from 5 to 7, characterized in that they correspond to the following formula:

Asn-Thr-Phe-Tyr
Asn-Thr-Phe-Tyr-Cys
Cys-Cys-Asn-Pro-Ala-Cys
Cys-Cys-Tyr-Pro-Ala-Cys
Asn-Thr-Phe-Tyr,Cys-Cys-Glu
Asn-Thr-Phe-Tyr-Cys-Cys-Glu-Leu
Cys-Cys-Gly-Leu-Cys-Cys-Asn-Pro-Ala-Cys-Ala-Gly-Cys
Cys-Cys-Gly-Leu-Cys-Cys-Tyr-Pro-Ala-Cys-Ala-Gly-Cys
Asn-Thr-Phe-Tyr-Cys-Cys-Gly-Leu-Cys-Cya-Asn-Pro-Ala-Cys
Ala-Gly-Cys-Tyr (1)
Asn-Thr-Phe-Tyr-Cys-Cys-Gly-Leu-Cys-Cys-Tyr,Pro-Ala-Cys
Ala-Gly-Cys-Asn (2)
Asn-Thr-Phe-Tyr-Cys-Gly-Gly-Gly (3)

11. Conjuguates characterized in that they areconstituted by a peptide according to anyone of claims 1 to 10, in association with a physiologically acceptable and non-toxic carrier molecule, preferably a protein advantageously selected from among ovalbumin, tetanus toxin, choleragenoid and Shigella cytotoxin.

12. Application of a peptide according to anyone of claims 1 to 11, to the manufacture of antibodies enabling the demonstration of human or animal ST toxins, particularly of pigs.

13. Application of a peptide according to anyone of claims 1 to 11, to the manufacture of antibodies enabling the vaccination of man or animals with respect to human or animal ST enterotoxins, particularly of pigs.

RAPPORT POIDS DE L'INTESTIN/POIDS DU CORPS

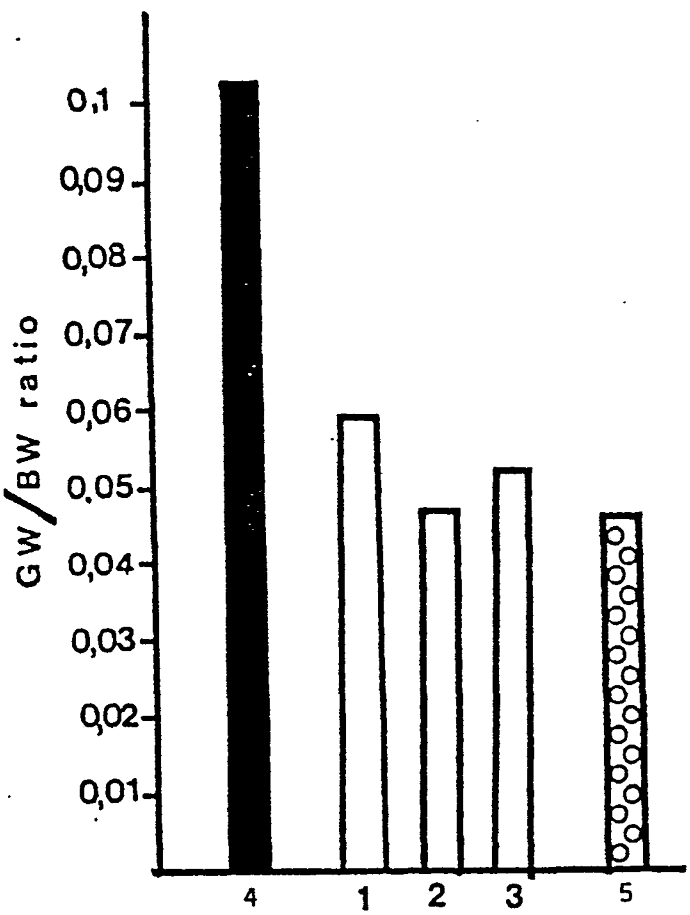


FIG.1.

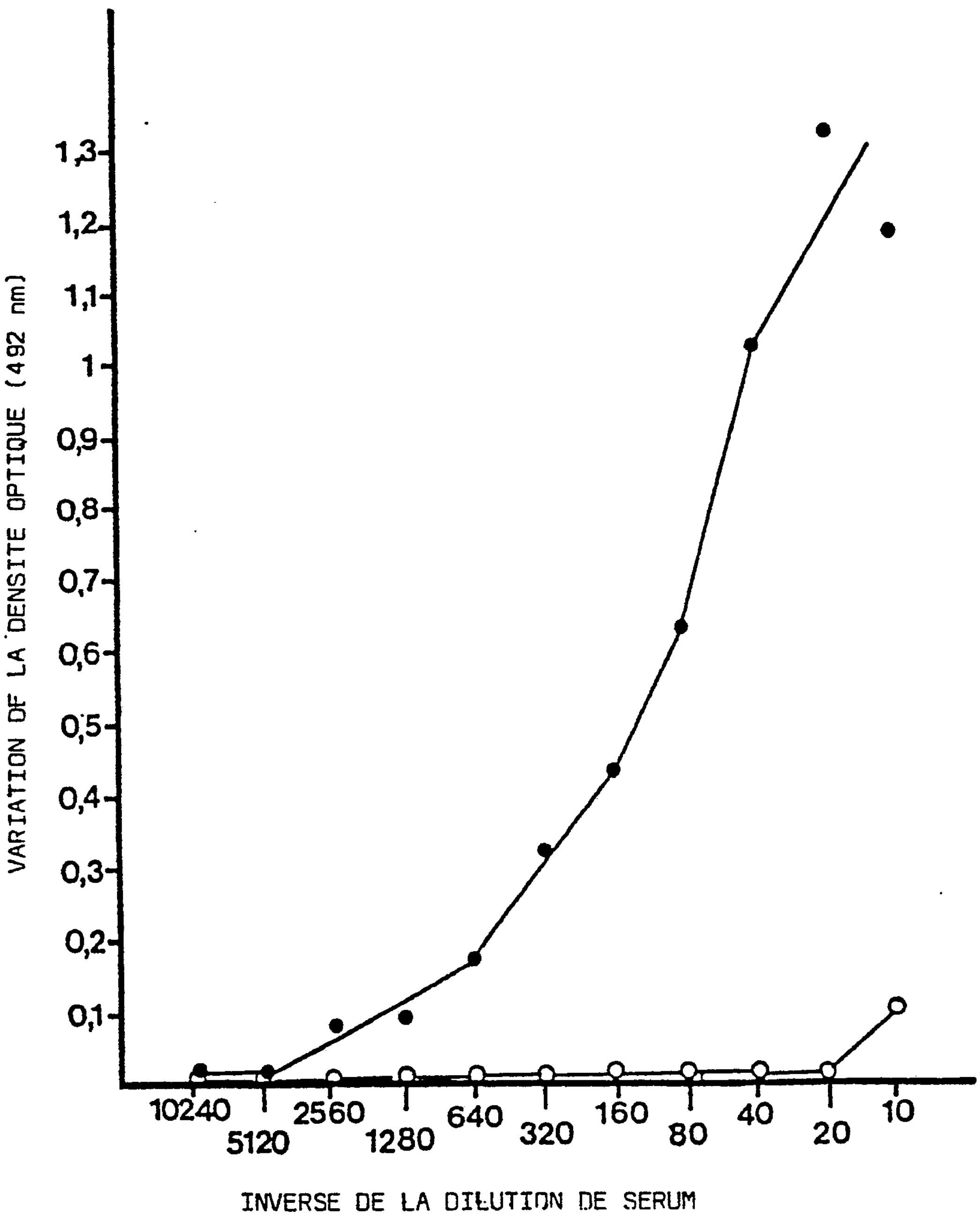

VARIATION DE LA DENSITE OPTIQUE (492 nm)
1,3
1,2
1,1
1
0,9
0,8
0,7
0,6
0,5
0,4
0,3
0,2
0,1
10240
5120
2560
1280
640
320
160
80
40
20
10
INVERSE DE LA DILUTION DE SERUM


FIG.2.

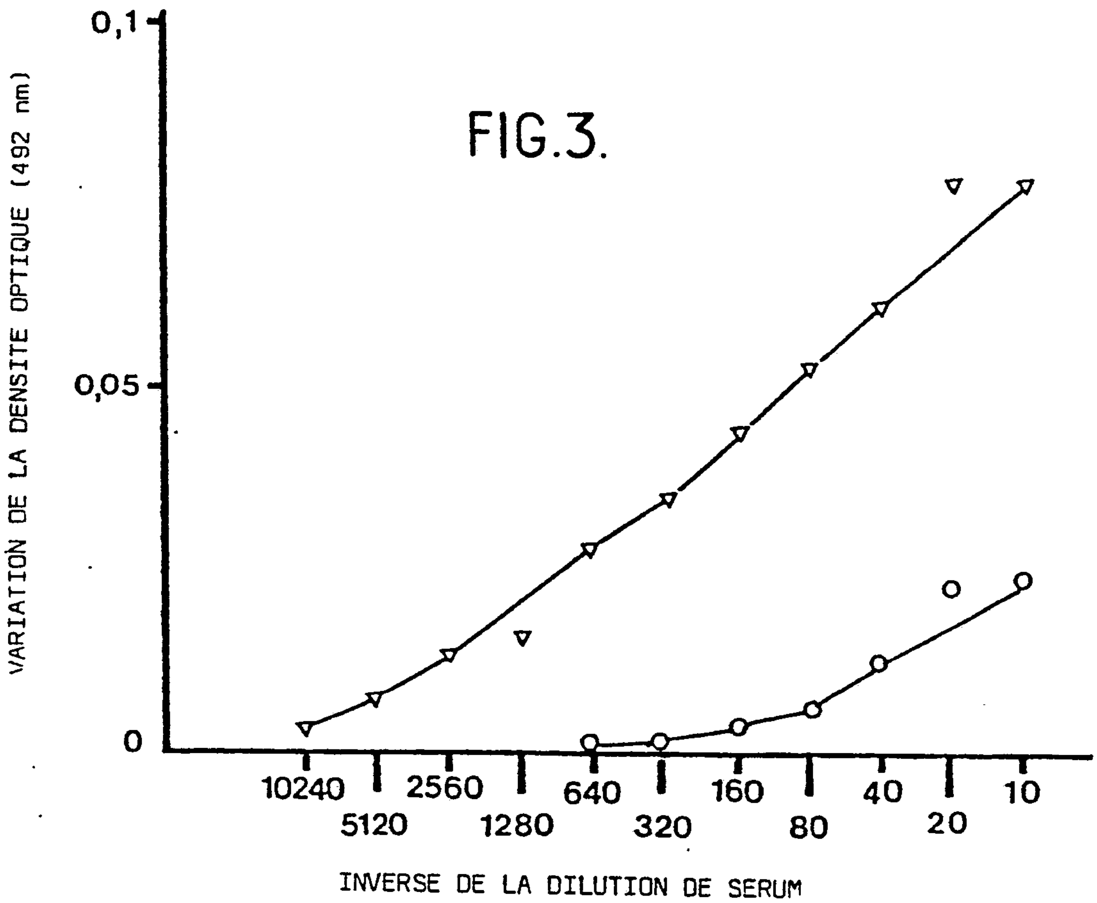
FIG.3.
VARIATION DE LA DENSITE OPTIQUE (492 nm)
0,1
0,05
0
10240
5120
2560
1280
640
320
160
80
40
20
10
INVERSE DE LA DILUTION DE SERUM

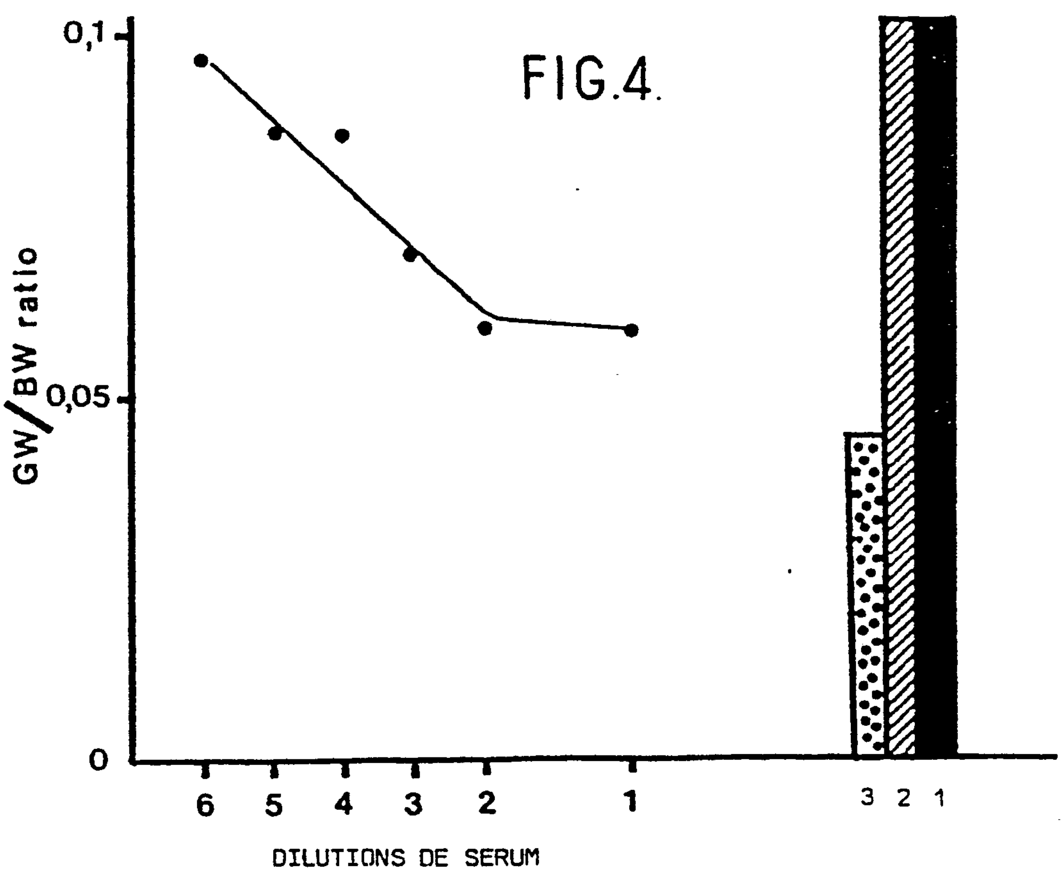
FIG.4.
GW/BW ratio
0,1
0,05
0
6
5
4
3
2
1
3 2 1
DILUTIONS DE SERUM

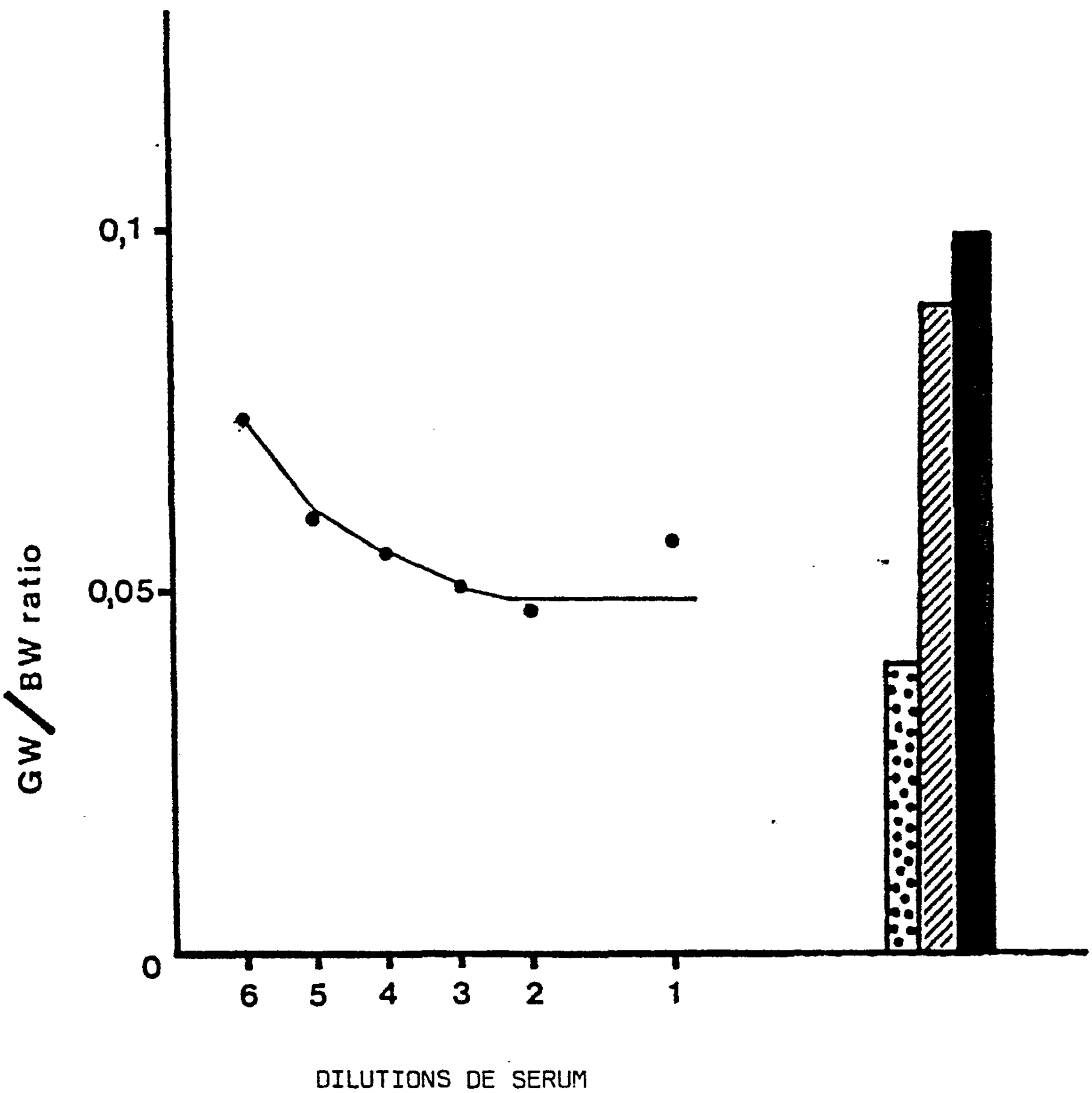


FIG.5.